# EUROPEAN PATENT APPLICATION

(11) **EP 4 790 152 A1**
(43) Date of publication of application: **12.08.2026**
(21) Application number: 24876458.1
(22) Date of filing: 30.09.2024
(51) Int. Cl.: C12Q 1/70, C12Q 1/686, C12N 15/11

(54) **PRIMER-PROBE COMPOSITION, NUCLEIC ACID DETECTION METHOD AND USE**

(30) Priority: 08.10.2023 CN 202311295503
(71) Applicant: Maccura Biotechnology Co., Ltd., Chengdu Sichuan 611731 (CN)
(72) Inventor: CHEN, Chao, Chengdu, Sichuan 611731 (CN); HU, Waner, Chengdu, Sichuan 611731 (CN); ZHAO, Yuhang, Chengdu, Sichuan 611731 (CN)
(74) Representative: Petraz, Gilberto Luigi
(86) International application number: PCT/CN2024/122731
(87) International publication number: WO 2025/077643

(57) **Abstract**

The present invention relates to the technical field of molecular biology. Provided are a primer-probe composition, a nucleic acid detection method and use thereof. The first primer comprises, in 5'-3' direction, a first target sequence binding region; the second primer comprises, in 5'-3' direction, a primer signal detection region, a second target sequence binding region and a third target sequence binding region; and the probe comprises a probe signal detection region. A complementary sequence of the primer signal detection region specifically binds to the probe signal detection region. When the primer-probe composition is used for nucleic acid detection, the false positive problem caused by the formation of dimers between primers can be effectively avoided, and the specificity and accuracy of multiplex nucleic acid detection is significantly improved.

## Description

### Field of the Invention

The present application relates to the technical field of molecular biology, and relates to a primer-probe composition, a nucleic acid detection method and use thereof.

### Priority information

The present application claims priority to Chinese patent application CN202311295503.1, filed on October 8, 2023, the content of which is incorporated herein by reference in its entirety.

### Background of the Invention

Polymerase chain reaction (PCR) is a molecular biological technology for enzymatic replication of DNA without using living organisms. The PCR is usually used in medical and biological research laboratories to undertake a variety of tasks, such as diagnosis of infectious diseases, gene cloning, phenotypic identification of laboratory animals, research on transcriptomes, detection of genetic diseases, identification of gene fingerprints, and paternity test. Since the PCR has an incomparable replication ability and accuracy, it is considered by the molecular biologists as a preferred method for nucleic acid detection. In the late 1990s, a real-time fluorescence quantitative PCR (qPCR) technology and related products launched by ABI in the USA further developed PCR into a highly sensitive, highly specific and accurately quantitative nucleic acid sequence analysis technology.

At present, a primer-probe design method that is widely used in qPCR platforms is mainly the TaqMan hydrolysis probe method, a working principle of which is mainly characterized by using an oligonucleotide probe that can specifically bind to a template and is labeled with a fluorescence group (donor) and a quencher group (receptor) at two ends thereof, and by designing a specific PCR primer upstream and downstream of the probe respectively. Before the start of PCR reaction, due to the principle of fluorescence resonance energy transfer (FRET), a fluorescence signal emitted by the fluorescence group at one end of the TaqMan probe is absorbed by the quencher group at the other end, such that the fluorescence signal cannot be detected by an instrument; but after the start of PCR amplification, the TaqMan probe specifically binds with the template, and when a DNA polymerase (Taqase) extends to a site at which the probe binds to the template, the TaqMan probe will be cleaved due to 5'-3' exonuclease activity of the Taqase, such that the fluorescence group labeled on the probe will be far away from the quencher group and a FRET structure cannot be formed any more, and thus a signal emitted by the fluorescence group can be detected by the instrument. However, to achieve PCR detection of multiple targets in the same reaction system, it is required to arrange multiple TaqMan hydrolysis probes labeled with fluorescence groups of different wavelengths, only a detection of 4-6-plex different targets can be achieved at most depending on a number of fluorescent channels in a detection instrument.

In order to achieve a higher level of multiplex detection, melting curve analysis is a good solution. Melting curve analysis is performed as follows: after an amplification reaction is completed, a melting curve is generated by gradually increasing temperature and monitoring a fluorescence signal at each step, wherein there is a characteristic peak corresponding to the melting temperature (Tm, a temperature at which a DNA double strand is melt by 50%), which may be used to distinguish a specific product from other products, even if they use the same fluorescence channel.

However, in the melting curve analysis, there presents a series of challenges for the design of primers and probes. Firstly, it is necessary to avoid non-specific amplification, which may result in false positive results due to the generation of melting temperatures similar to the melting temperature of the specific product. Secondly, the formation of primer dimers may generate interference signals, thereby affecting the accuracy of analysis. In addition, in order to ensure that different targets may be accurately distinguished by means of the melting curve, there must be a sufficient difference between the melting temperatures of different targets for identification.

### Summary of the Invention

The present application provides a primer-probe composition, a nucleic acid detection method, and the use thereof. A second primer of the primer-probe composition is partially self-complementary in sequence and forms a stem-loop structure in the absence of a target, thereby avoiding the false positive problem caused by the formation of primer dimers between multiple pairs of primers during amplification; and when a target is present, the stem-loop structure of the primer will melt, and the primer binds specifically to the target for a first amplification. One specific amplification product formed after the first amplification is a single-stranded amplification product (S1), which is capable of forming a new stem-loop structure with its 3' end released, so as to carry out a second amplification that can cause a signal change. However, a non-specific amplification product cannot form a new stem-loop structure, and it has a stem-loop structure similar to that of the second primer, and its 3' end is blocked due to the stem formation, making it difficult to carry out a subsequent specific amplification that can cause a signal change. Therefore, the specificity of the amplification process has been significantly improved. When the probes and primers are designed, the probe can be completely unrelated or non-strictly complementary to the target sequence, making the probe design simpler, more convenient, and more flexible. The probe can specifically bind to a part of the complementary sequences of the second primer, thus by adjusting the length and position of the specifically binding sequences between them, amplification products formed with the probe with different Tm values can be obtained; and then nucleic acid detection can be achieved by judging whether the target is present according to the Tm values.

Therefore, the present application can solve the false positive problem caused by non-specific amplification of primers in the presence of multiple specific primers in a single tube reaction. Furthermore, the target source comprises bacteria, viruses, human genomes, etc. such that clinical units, including primary hospitals, can quickly carry out common nucleic acid detection, such as pathogenic microorganism infection detection, thereby, in combination with other detection and confirmed means, providing diagnostic evidences and medication schemes for clinical practice more quickly, reducing the patient's mental and economic burdens, and achieving the objective of precision medicine.

The present application involves the following:
1. A primer-probe composition for nucleic acid detection, comprising at least one first primer, at least one second primer, and at least one probe;
   the first primer comprises, from 5' end to 3' end, a first target sequence binding region (X1');
   the second primer comprises, from 5' end to 3' end, a primer signal detection region (h), a second target sequence binding region (X2'), and a third target sequence binding region (X3'); when a target is not present, the primer signal detection region (h) specifically binds to a partial sequence of the second primer to form a first stem-loop structure;
   wherein, the first target sequence binding region (X1') and the second target sequence binding region (X2') specifically bind to a first target sequence (X1) and a second target sequence (X2) of one target chain in a double-stranded target respectively, and the third target sequence binding region (X3') can specifically bind to a third target sequence (X3) of the other target chain in the double-stranded target;
   the probe comprises a probe signal detection region (H) and a first detection group and a second detection group labeled at any position on the probe, wherein the first detection group and the second detection group generate a detectable signal change through a change in distance between them on the probe; and
   a complementary sequence of the primer signal detection region (h) specifically binds to the probe signal detection region (H).
2. The primer-probe composition of item 1, wherein, when a target is not present, the partial sequence of the second primer that specifically binds to the primer signal detection region (h) to form the first stem-loop structure is a complementary sequence (h') of the primer signal detection region, which is located between the second target sequence binding region (X2') and the third target sequence binding region (X3');
   the primer signal detection region (h), the complementary sequence (h') of the primer signal detection region, and the probe are not complementary and/or identical to any target sequence; and
   preferably, the second primer further comprises a third linker sequence located between the complementary sequence (h') of the primer signal detection region and the third target sequence binding region (X3'), wherein the third linker sequence is not complementary and/or identical to any target sequence.
3. The primer-probe composition of item 1, wherein, when a target is not present, the partial sequence of the second primer that specifically binds to the primer signal detection region (h) to form the first stem-loop structure is the second target sequence binding region (X2'), and the second primer further comprises a third linker sequence located between the second target sequence binding region (X2') and the third target sequence binding region (X3'), wherein the third linker sequence is not complementary and/or identical to any target sequence.
4. The primer-probe composition of any one of items 1-3, wherein any two of the first target sequence binding region (X1'), the third target sequence binding region (X3'), and the second target sequence binding region (X2') are not complementary or identical in sequence.
5. The primer-probe composition of any one of items 1-4, wherein the first primer and the second primer each independently have a length of 20-80 bases.
6. The primer-probe composition of any one of items 1-5, wherein the first target sequence binding region (X1') has a length of 15-45 bases; and/or, the first target sequence binding region (X1') has a Tm value of 40-80°C.
7. The primer-probe composition of any one of items 1-6, wherein the primer signal detection region (h) and the complementary sequence (h') of the primer signal detection region each independently have a length of 5-65 bases; and/or, the primer signal detection region (h) and the complementary sequence (h') of the primer signal detection region each independently have a Tm value of 30-80 °C.
8. The primer-probe composition of any one of items 1-7, wherein the second target sequence binding region (X2') has a length of 5-65 bases; and/or, the second target sequence binding region (X2') has a Tm value of 40-80 °C.
9. The primer-probe composition of any one of items 1-8, wherein the third target sequence binding region (X3') has a length of 5-65 bases; and/or, the third target sequence binding region (X3') has a Tm value of 40-80 °C.
10. The primer-probe composition of any one of items 1-9, wherein the probe signal binding region (H) has a length of 5-65 bases; and/or, the probe signal binding region (H) has a Tm value of 30-85 °C.
11. The primer-probe composition of any one of items 1-10, wherein the first primer further comprises a probe anchoring region (A) located at 5' end of the first target sequence binding region (X1'); the probe anchoring region (A) is used to bind to the probe; and the probe anchoring region (A) is not complementary and/or identical to any target sequence.
12. The primer-probe composition of item 11, wherein the probe anchoring region (A) has a length of 5-35 bases; and/or, the probe anchoring region (A) has a Tm value of 40-80 °C; and/or, the probe anchoring region (A) has a GC content of 40-80%.
13. The primer-probe composition of item 11 or 12, wherein the probe further comprises a primer anchoring region (A'); the probe anchoring region (A) is complementary to the primer anchoring region (A') in sequence.
14. The primer-probe composition of item 13, wherein the primer anchoring region (A') has length of 5-35 bases; and/or, the primer anchoring region (A') has a Tm value of 40-80 °C; and/or, the primer anchoring region (A') has a GC content of 40-85%.
15. The primer-probe composition of any one of items 1-14, wherein the probe comprises, from 5' end to 3' end, the probe signal detection region (H) and the primer anchoring region (A');
   or, the probe comprises, from 3' end to 5' end, the probe signal detection region (H) and the primer anchoring region (A').
16. The primer-probe composition of any one of items 13-15, wherein a Tm value of the probe anchoring region (A) is greater than a Tm value of the primer signal detection region (h).
17. The primer-probe composition of any one of items 11-14 and 16, wherein the first primer further comprises a fourth linker sequence located between the probe anchoring region (A) and the first target sequence binding region (X1');
   and/or, the second primer further comprises a first linker sequence located between the primer signal detection region (h) and the second target sequence binding region (X2'),
   and/or, a second linker sequence located between the second target sequence binding region (X2') and the complementary sequence (h') of the primer signal detection region;
   and/or, the probe further comprises a fifth linker sequence located between the primer anchoring region (A') and the probe signal detection region (H); and
   the first linker sequence, the second linker sequence, the fourth linker sequence, and the fifth linker sequence each are not complementary and/or identical to any target sequence.
18. The primer-probe composition of any one of items 1-17, wherein the first detection group is a fluorescence reporter group, and the second detection group is a fluorescence quencher group or a group that produces a fluorescence signal with the fluorescence reporter group via fluorescence resonance energy transfer;
   preferably, the fluorescence reporter group is any one or more selected from ALEX-350, FAM, VIC, TET, CAL FluorGold540, JOE, HEX, CAL Fluor Orange 560, TAMRA, CAL Fluor Red 590, ROX, CAL Fluor Red 610, TEXAS RED, CAL Fluor Red 635, Quasar 670, Cy3, Cy5, Cy5.5 and Quasar 705; and the fluorescence quencher group is any one or more selected from DABCYL, BHQ class (such as BHQ-1 or BHQ-2), ECLIPSE, and TAMRA; and
   preferably, on the probe, a separation distance between the fluorescence reporter group and the fluorescence reporter group is 3-250 angstroms; preferably, 3-201 angstroms; and more preferably, 3-140 angstroms.
19. The primer-probe composition of any one of items 1-18, wherein the probe has a blocking region at 3' end thereof.
20. The primer-probe composition of any one of items 1-19, wherein, when the primer-probe composition comprises one probe and at least two second primers, sequences of the primer signal detection regions (h) on different second primers are different, and positions where the complementary sequences of the primer signal detection regions (h) on different second primers specifically bind to the probe signal binding region (H) are different; and
   preferably, one, two or more first primer(s) is/are included.
21. A primer-probe system for nucleic acid detection, comprising the primer-probe composition of any one of items 1-20.
22. A nucleic acid detection method, comprising contacting a target sample with the primer-probe composition of any one of items 1-20 and/or the primer-probe system of item 1, performing at least two amplifications, and analyzing whether a target is present;
   wherein, the target sample includes a target sequence;
   preferably, a source of the target sample includes any one or more of bacteria, viruses, and pathogens;
   preferably, a method for analyzing whether a target is present comprises: determining whether the target is present by a signal change before and after amplification; and/or, determining whether the target is present by a melting curve analysis of an amplification product; and/or, determining whether the target is present by a signal change before and after denaturation of the amplification product; and
   preferably, the amplification comprising: randomly allocating a reaction system to at least 500 reaction units, wherein each reaction unit contains a target of a sample to be detected or contains no target of the sample to be detected;
   and/or, performing PCR amplification on all the reaction units.
23. The method of item 22, wherein after the target sample is contacted with the primer-probe composition and/or the primer-probe system, when the target is present in the target sample, a first specific amplification is performed by taking the target as a template and the first primer and second primer as primers to obtain a single-stranded amplification product (S1); wherein, the single-stranded amplification product (S1) comprises, from 5' end to 3' end, at least the first target sequence binding region (X1'), a complementary sequence of the second target sequence (X2), a complementary sequence (X3") of the third target sequence binding region (X3'), optionally a complementary sequence of the third linker sequence and/or a complementary sequence of the complementary sequence (h') of the primer signal detection region, a complementary sequence (X2") of the second target sequence binding region (X2'), and the complementary sequence of the primer signal detection region (h);
   in the single-stranded amplification product (S1), a Gibbs free energy of a conformation formed by specific binding of the complementary sequence of the second target sequence (X2) and the complementary sequence (X2") of the second target sequence binding region (X2') is lower than a Gibbs free energy of a conformation formed by specific binding of the complementary sequence of the primer signal detection region (h) and the complementary sequence of the complementary sequence (h') of the primer signal detection region; or, the Gibbs free energy of the conformation formed by specific binding of the complementary sequence of the second target sequence (X2) and the complementary sequence (X2") of the second target sequence binding region (X2') is lower than a Gibbs free energy of conformation formed by specific binding of the complementary sequence of the primer signal detection region (h) and the complementary sequence (X2") of the second target sequence binding region (X2');
   the single-stranded amplification product (S1) forms a second stem-loop structure by binding the complementary sequence of the second target sequence to the complementary sequence (X2") of the second target sequence binding region (X2');
   when the target is present, the second stem-loop structure of the single-stranded amplification product (S1) is melt, and a second specific amplification is performed by taking the probe as a template and the single-stranded amplification product (S1) as a primer to obtain a double-stranded amplification product formed with the probe; and
   a melting temperature of the double-stranded amplification product formed with the probe is detected to achieve nucleic acid detection.
24. Use of any one of the primer-probe compositions of items 1-20 and/or the primer-probe system of item 21, comprising preparation of a product for nucleic acid detection and/or nucleic acid detection;
   preferably, the product for nucleic acid detection is associated with a disease; and
   more preferably, the disease is papilloma.
25. A kit for nucleic acid detection, comprising the primer-probe composition of any one of items 1-20 and/or the primer-probe system of item 21;
   preferably, the kit further comprises an amplification reagent;
   more preferably, the amplification reagent comprises DNA polymerase and dNTPs; and
   more preferably, the amplification reagent further comprises a reverse transcriptase.

### Advantageous effect

1. Multiplex detection: the method described in the present invention can simultaneously perform analysis in two dimensions of a fluorescence channel and a melting temperature in a single tube reaction, that is, by using a same fluorescence channel, different targets can be detected by means of melting temperature characteristics; or by using different fluorescent channels, a target type detection of the product of the number of the fluorescent channels and the melting temperature characteristic can be achieved.
2. Strong specificity: in the method described in the present application, the primer may be blocked to form a hairpin structure (i.e., the first stem-loop structure formed by the second primer) when a target sequence is not present; and the second primer has two target sequence binding regions (i.e., the second target sequence binding region (X2') and the third target sequence binding region (X3')); and when a target sequence is present, the previous hairpin structure of the second primer will be opened, which will not affect amplification efficiency, and the resulting single-stranded amplification product will form another hairpin structure, which will not affect the binding with the probe to generate a signal.
3. High sensitivity: according to the method described in the present application, the probe can be completely unrelated to the target sequence, such that the requirement for the length of the target sequence is very low; and when the target is a type of short-fragment nucleic acid, such as a free nucleic acid, a higher sensitivity will be achieved for detection of a target sequence with a shorter length.
4. Low fluorescence background: for each fluorescence channel, only one probe is used, such that not only the reagent cost is reduced, but also a differentiation can be achieved through the different melting temperatures of the amplification products, thereby greatly reducing the fluorescence background in the PCR reaction and improving reaction sensitivity.
5. Adjustable melting temperature: in the method described in the present application, a differentiation is achieved by using the different melting temperatures of the double-stranded secondary amplification products formed with the probe, such that the melting temperatures of the secondary amplification double-stranded products formed with the probe may be increased or decreased by adjusting a length or sequence of the primer signal detection region (h), and/or adjusting a position at which the complementary sequence of the primer signal detection region (h) specifically binds to the probe signal detection region (H) of the probe. This generates a sufficient difference in melting temperatures for different targets, and different targets can be easier to be identified accurately.
6. Good inclusiveness: compared with the Taqman hydrolysis probe method which requires three parts (first primer, second primer and probe) to complementarily pair with the template, in the primer-probe design method of the present invention, only two parts, namely the first primer and the second primer, are required to complementarily pair with the target sequence. When the target sequence has many hypervariable regions, such as viral or bacterial genomes, the primer-probe design solution of the present application has a better inclusiveness and a lower design difficulty.
7. Wide applicable range: the method described in the present application is suitable for nucleic acid detection of various sample types, including a serum sample, a plasma sample, a whole blood sample, a sputum sample, a swab sample, a lavage fluid sample, a fresh tissue sample, a formalin fixed paraffin embedded tissue (FFPE).

### Brief Description of the Drawings

Fig. 1 is a melting curve of amplification product after using reverse primer (R1) in Example 2.
Fig. 2 is a melting curve of amplification product after using reverse primer (R2) in Example 2.
Fig. 3 is a melting curve of amplification product after using reverse primer (R3) in Example 2.
Fig. 4 shows melting curves of four targets in Example 3.
Fig. 5 is a melting curve of a template-free control when the primers and probe were added simultaneously in Example 3, with an abscissa representing temperature (°C).

### Detailed Description of the Embodiments

It should be noted that certain vocabularies are used in the specification and claims to refer to specific components. It may be understood by those skilled in the art that different terms may be used to refer to the same component. The specification and claims do not distinguish components based on differences in terminology, but rather based on functional differences of the components.

As the term "include" or "comprise" mentioned throughout the specification and claims is an open-ended term, it should be interpreted as "including but not limited to". The subsequent description in the specification involves preferred embodiments for implementing the present application, which are provided for the general principles of the specification and are not intended to limit the scope of the present application. The scope of protection of the present application shall be as defined by the appended claims. Unless otherwise defined, all the technical and scientific terms used herein have the same meanings as those commonly understood by those skilled in the art to which the present disclosure pertains.

It should be understood that the embodiments of the present application described herein comprise embodiments involving "consist of..." and/or "essentially consist of...". The reference to "about" preceding a value or parameter herein comprises (and describes) variations in the value or parameter itself. For example, the reference to the description of "approximately X" includes a description of "X".

As used herein, the reference to "not" preceding a value or parameter typically means and describes values or parameters "other than". For example, where a method is not used for treating X-type cancer, it means that the method is used for treating cancers other than the X-type cancer.

As used herein, the term 'approximately X-Y' has the same meaning as' approximately X to approximately Y'.

As used herein and in the appended claims, the singular forms "a", "an" and "the" include plural references unless the context clearly dictates otherwise. It should also be noted that the claim may be drafted to exclude any optional elements. Therefore, the statement is intended to serve as a antecedent basis for the use of exclusive terms such as "only", "merely" in combination with the description of the elements in the claims, or for the use of "no" as limitation.

As used herein, the terms "and/or" in phrases such as "A and/or B" is intended to include both A and B; A or B; A (alone); and B (alone). Likewise, the term "and/or" when used in phrases such as "A, B, and/or C" is intended to include the following embodiments: A, B, and C; A, B, or C; A or C; A or B; B or C; A and C; A and B; B and C; A (alone); B (alone); and C (alone).

As used herein, the term "each independently" means that the selections (such as selection of values) of the various factors are unrelated and uncorrelated. For example, the expression "A and B are each independently m, n, q" means that when A is m, B may be m, n, or q; for example, when A is n, B may be m, n, or q; or for example, when B is q, A may be m, n, or q.

As used herein, "specifically bind in a paired manner" or "specifically bind" refers to complementary base pairing and binding of two single-stranded nucleic acid molecules with complementary sequences under certain conditions (such as appropriate temperature and ionic strength). Those skilled in the art are well known that, even if two single-stranded nucleic acid molecules are not completely complementary and paired, they still may specifically bind, that is, they can also specifically bind in the presence of a few base mismatches.

A primer-probe composition for nucleic acid detection, comprises at least one first primer, at least one second primer, and at least one probe; wherein,
the first primer comprises, from 5' end to 3' end, a first target sequence binding region (X1');
the second primer comprises, from 5' end to 3' end, a primer signal detection region (h), a second target sequence binding region (X2'), and a third target sequence binding region (X3'); and when a target is not present, the primer signal detection region (h) specifically binds to a partial sequence of the second primer to form a first stem-loop structure;
wherein, the first target sequence binding region (X1') and the second target sequence binding region (X2') specifically bind to a first target sequence and a second target sequence of one target chain in a double-stranded target respectively, and the third target sequence binding region (X3') can specifically bind to a third target sequence of the other target chain in the double-stranded target;
the probe comprises a probe signal detection region (H) and a first detection group and a second detection group labeled at any position on the probe, wherein the first detection group and the second detection group generate a detectable signal change through a change in distance between them on the probe; and
a complementary sequence of the primer signal detection region (h) specifically binds to the probe signal detection region (H).

In the present application, "complementary" refers to forward complementary or reverse complementary; and preferably reverse complementary.

It should be noted that, both the first target sequence binding region (X1') and the third target sequence binding region (X3') are target-specific sequences. The target-specific sequence refers to a specific region used for specific recognition and amplification of a target DNA or RNA sequence in PCR (polymerase chain reaction) or other molecular biology experiments. For the amplification of a double-stranded target, the first target sequence binding region (X1') and the second target sequence binding region (X2') can bind to the same target chain in the double-stranded target (one target chain in the double-stranded target, i.e., a target single chain); and the third target sequence binding region (X3') is capable of binding to the other target chain in the double-stranded target (one target chain in the double-stranded target, i.e. a target single chain). For example, the double-stranded target comprises a target single chain A and a target single chain B, wherein the first target sequence binding region (X1') and the second target sequence binding region (X2') bind to different fragments of target single chain A, that is, the target single chain A comprises a first target sequence (X1) and a second target sequence (X2) thereon; and the third target sequence binding region (X3') binds to the target single chain B, that is, the target single chain B comprises a third target sequence (X3) thereon.

The target in the present application is a double-stranded DNA. The target may be a double-stranded DNA obtained from an original double-stranded DNA, or a double-stranded DNA obtained by amplification of a single-stranded DNA, or a double-stranded DNA obtained by reverse transcription from a RNA; and the target may exist naturally, may be obtained through engineering modification, or may be artificially synthesized.

In some embodiments, the first primer is a forward primer and the second primer is a reverse primer; and in some embodiments, the first primer is a reverse primer and the second primer is a forward primer.

In some embodiments, when a target is not present, the partial sequence of the second primer that specifically binds to the primer signal detection region (h) to form the first stem-loop structure is a complementary sequence (h') of the primer signal detection region, which is located between the second target sequence binding region (X2') and the third target sequence binding region (X3'); the primer signal detection region (h), the complementary sequence (h') of the primer signal detection region, and the probe are not complementary and/or identical to any target sequence. In some embodiments, the sequences of the primer signal detection region (h) and the complementary sequence (h') of the primer signal detection region are forward or reverse complementary; and preferably are reverse complementary. In some embodiments, the second primer further comprises a first linker sequence located between the primer signal detection region (h) and the second target sequence binding region (X2'). In some embodiments, the second primer further comprises a second linker sequence located between the second target sequence binding region (X2') and the complementary sequence (h') of the primer signal detection region. In some embodiments, the second primer further comprises a third linker sequence located between the complementary sequence (h') of the primer signal detection region and the third target sequence binding region (X3'). Wherein, the first linker sequence, the second linker sequence, and the third linker sequence each are not complementary and/or identical to any target sequence. In some embodiments, the second primer comprises, from 5' end to 3' end, the primer signal detection region (h), the second target sequence binding region (X2'), the complementary sequence (h') of the primer signal detection region, the third linker sequence, and the third target sequence binding region (X3'). In some embodiments, the second primer comprises, from 5' end to 3' end, the primer signal detection region (h), the second target sequence binding region (X2'), the complementary sequence (h') of the primer signal detection region, and the third target sequence binding region (X3'). In some embodiments, the second primer comprises, from 5' end to 3' end, the primer signal detection region (h), the first linker sequence, the second target sequence binding region (X2'), the complementary sequence (h') of the primer signal detection region, the third linker sequence, and the third target sequence binding region (X3'). In some embodiments, the second primer comprises, from 5' end to 3' end, the primer signal detection region (h), the second target sequence binding region (X2'), the second linker sequence, the complementary sequence (h') of the primer signal detection region, the third linker sequence, and the third target sequence binding region (X3'). In some embodiments, the second primer comprises, from 5' end to 3' end, the primer signal detection region (h), the first linker sequence, the second target sequence binding region (X2'), the second linker sequence, the complementary sequence (h') of the primer signal detection region, and the third target sequence binding region (X3'). In some embodiments, the second primer comprises, from 5' end to 3' end, the primer signal detection region (h), the first linker sequence, the second target sequence binding region (X2'), the second linker sequence, the complementary sequence (h') of the primer signal detection region, the third linker sequence, and the third target sequence binding region (X3').

In some embodiments, the Tm value of the primer signal detection region (h) is lower than that of the first target sequence binding region (X1') and that of the third target sequence binding region (X3') in the first primer, such that during the nucleic acid detection process, the first target sequence binding region (X1') and the third target sequence binding region (X3') first bind to the target for a first amplification, forming a single-stranded amplification product (S1), and then the complementary sequence of the primer signal detection region (h) is released and specifically binds to the probe signal detection region (H) on the probe.

When a sequence amplification is performed, when a target sequence is not present in the amplification system or the content the of the target sequence is too low to perform amplification, the primer signal detection region (h) specifically binds to the complementary sequence (h') of the primer signal detection region, such that the second primer forms a stem-loop structure. Specifically, the primer signal detection region (h) forms a "stem" with the complementary sequence (h') of the primer signal detection region, while the second target sequence binding region (X2') located between the primer signal detection region (h) and the complementary sequence (h') of the primer signal detection region forms a "loop". The stem-loop structure avoids the false positive problem caused by the formation of primer dimers between primers. When a target is present in the amplification system, by taking the target as a template, the second primer extends in a 3' direction of the third target sequence binding region (X3') to form an extended chain, on which a second target sequence (X2) that can specifically bind to the second target sequence binding region (X2') is included, and the binding of the second target sequence (X2) and the second target sequence binding region (X2') disrupts the "stem" formed by the primer signal detection region (h) and the complementary sequence (h') of the primer signal detection region.

In some embodiments, when the target is absent, a partial sequence of the second primer that binds to the primer signal detection region (h) to form the first stem-loop structure is the second target sequence binding region (X2'). The second primer further comprises a third linker sequence located between the second target sequence binding region (X2') and the third target sequence binding region (X3'), wherein the third linker sequence is not complementary and/or identical to any target sequence, the binding between the primer signal detection region (h) and the second target sequence binding region (X2') is not a completely base-complementary binding, the primer signal detection region (h) and the second target sequence binding region (X2') involves partial sequence complementarity. For example, a partial sequence of the primer signal detection region (h) is complementary to a partial sequence of the second target sequence binding region (X2'); or, an entire sequence of the primer signal detection region (h) is complementary to a partial sequence of the second target sequence binding region (X2'); or, a partial sequence of the primer signal detection region (h) is complementary to an entire sequence of the second target sequence binding region (X2').

In some embodiments, the second primer further comprises a first linker sequence located between the primer signal detection region (h) and the second target sequence binding region (X2'). In some embodiments, the second primer further comprises a second linker sequence located between the second target sequence binding region (X2') and the complementary sequence (h') of the primer signal detection region. In some embodiments, the second primer comprises, from 5' end to 3' end, the primer signal detection region (h), the second target sequence binding region (X2'), the third linker sequence that is not complementary and/or identical to any target sequence, and the third target sequence binding region (X3'). In some embodiments, the primer signal detection region (h) and the second target sequence binding region (X2') in the second primer are directly connected; at this point, the sequence of the primer signal detection region (h) is long enough to form the "stem" part in the stem-loop structure, and a partial sequence of the primer signal detection region (h) is complementary to a partial sequence of the second target sequence binding region (X2'). In some embodiments, the first linker sequence is connected between the primer signal detection region (h) and the second target sequence binding region (X2') of the second primer; at this point, the primer signal detection region (h) is relatively short, and the first linker sequence is connected between the primer signal detection region (h) and the second target sequence binding region (X2'), such that in the formed a stem-loop structure, the "loop" part is mainly formed by the first linker sequence. In some embodiments, the second primer comprises, from 5' end to 3' end, the primer signal detection region (h), the first linker sequence, the second target sequence binding region (X2'), the third linker sequence, and the third target sequence binding region (X3'). In some embodiments, the second primer comprises, from 5' end to 3' end, the primer signal detection region (h), the second target sequence binding region (X2'), the third linker sequence, and the third target sequence binding region (X3'). In some embodiments, the second primer comprises, from 5' end to 3' end, the primer signal detection region (h), the first linker sequence, the second target sequence binding region (X2'), the second linker sequence, the complementary sequence (h') of the primer signal detection region, the third linker sequence, and the third target sequence binding region (X3'). In some embodiments, the first primer is F1 having a sequence as shown in SEQ ID NO: 2. In some embodiments, by way of example, the second primer is R2 having a sequence as shown in SEQ ID NO: 4, or R3 having a sequence as shown in SEQ ID NO: 5.

When a sequence amplification is performed, when a target sequence is not present in the amplification system or the content of the target sequence is too low to perform amplification, the primer signal detection region (h) and the second target sequence binding region (X2') bind via complementary pairing of partial base sequences, causing the second primer to form a stem-loop structure. Specifically, a partial sequence of the primer signal detection region (h) complementarily binds to the second target sequence binding region (X2') to form a "stem", while the part of the primer signal detection region (h) that is completely not complementary to the second target sequence binding region (X2') forms a "loop". The stem-loop structure avoids the false positive problem caused by the formation of primer dimers between primers. Of course, it will be understood that, if the "loop" is only formed by the primer signal detection region (h), then the primer signal detection region (h) is sufficiently long. When a target is present in the amplification system, by taking one chain of the double-stranded target as a template, the second primer extends in a 3' direction of the third target sequence binding region (X3') to form an extended chain, on which the second target sequence (X2) is included, and the second target sequence (X2) specifically binds to the second target sequence binding region (X2'), causing the "stem" formed by the primer signal detection region (h) and the second target sequence binding region (X2') to be disrupted.

In some embodiments, the first primer further comprises a probe anchoring region (A) located at 5' end of the first target sequence binding region (X1'); the probe anchoring region (A) is used to bind to the probe; and the probe anchoring region (A) is not complementary and/or identical to any target sequence. In some embodiments, the first primer further comprises a fourth linker sequence located between the probe anchoring region (A) and the first target sequence binding region (X1'), wherein the fourth linker sequence is not complementary and/or identical to any target sequence. In some embodiments, the first primer comprises, from 5' end to 3' end, the probe anchoring region (A) and the first target sequence binding region (X1'). In some embodiments, the first primer comprises, from 5' end to 3' end, the probe anchoring region (A), the fourth linker sequence, and the first target sequence binding region (X1').

In some embodiments, the probe further comprises a primer anchoring region (A'); and the primer anchoring region (A') is not complementary and/or identical to any target sequence. In some embodiments, the primer anchoring region (A') is complementary to the probe anchoring region (A); in some embodiments, the primer anchoring region (A') is forward complementary to the probe anchoring region (A); and in some embodiments, the primer anchoring region (A') is reverse complementary to the probe anchoring region (A). In some embodiments, the probe further comprises a fifth linker sequence located between the primer anchoring region (A') and the probe signal detection region (H), wherein the fifth linker sequence is not complementary and/or identical to any target sequence.

It should be noted that the positioning of the anchoring region (A') and probe signal detection area (H) on the probe in the present application is not limited and may be set arbitrarily. In some embodiments, the probe comprises, from 5' end to 3' end, the probe signal detection region (H). In some embodiments, the probe comprises, from 5' end to 3' end, the primer anchoring region (A') and the probe signal detection region (H). Further, the first detection group and the second detection group are labeled between the primer anchoring region (A') and the probe signal detection region (H). In some embodiments, the probe comprises, from 5' end to 3' end, the primer anchoring region (A'), the fifth linker sequence, and the probe signal detection region (H). In some embodiments, the probe comprises, from 5' end to 3' end, the fifth linker sequence and the probe signal detection region (H). In some embodiments, the probe comprises, from 3' end to 5' end, the probe signal detection region (H). In some embodiments, the probe comprises, from 3' end to 5' end, the primer anchoring region (A') and the probe signal detection region (H). Further, the first detection group and the second detection group are labeled at 5' end of the probe signal detection region (H). In some embodiments, the probe comprises, from 3' end to 5' end, the primer anchoring region (A'), the fifth linker sequence, and the probe signal detection region (H). In some embodiments, the probe comprises, from 3' end to 5' end, the fifth linker sequence and the probe signal detection region (H).

In a specific example, the probe is probe P having a sequence as shown in SEQ ID NO: 1. In some embodiments, the primer-probe composition is P1F1R2. In some embodiments, the primer-probe composition is P1F1R3. In some embodiments, the primer-probe composition is P1F1R1R2. In some embodiments, the primer-probe composition is P1F1R1R3. In some embodiments, the primer-probe composition is P1F1R1R2R3. In some embodiments, the primer pair is P1F1R2R3.

In some embodiments, the probe comprises one or more probe signal detection regions (H). In some embodiments, the probe comprises one or more primer anchoring regions (A').

In some embodiments, the probe comprises 1-2 primer anchoring regions (A') and 1-2 probe signal detection regions (H).

In some embodiments of the present invention, the probe has any one structure of 1) to 4);
1) the probe comprising one primer anchoring region (A') and one probe signal detection region (H);
2) the probe comprising one probe signal detection region (H) and two primer anchoring regions (A'), wherein the two primer anchoring regions (A') are located on two sides of the probe signal detection region (H), respectively, and the two primer anchoring regions (A') are different in sequence;
3) the probe comprising one primer anchoring region (A') and two probe signal detection regions (H), wherein the two probe signal detection regions (H) are located on two sides of the primer anchoring region (A'), and the two probe signal detection regions (H) are different in sequence; and
4) the probe comprising two primer anchoring regions (A') and two probe signal detection regions (H), wherein the primer anchoring regions (A') and the probe signal detection regions (H) are arranged at intervals, the two primer anchoring regions (A') are different in sequence, and the two probe signal detection regions (H) are different in sequence.

In some embodiments, the probe comprises a blocking region at 3' end thereof. With this solution, during second amplification after binding of the single-stranded amplification product (S1) with the probe, the 3' end of the single-stranded amplification product (S1) only extends to the end of the probe, thereby reducing non-specific amplification.

In the present application, the blocking region is a portion that is configured to prevent extension of the nucleic acid chain through DNA polymerase, thereby blocking strand extension during, for example, a PCR process. The blocking region may be one where the 3'OH is labeled by 3'-Spacer C3, 3'-Phosphat, 3'-ddC, 3'-Inverted End, such that the 3' OH is blocked, so as to prevent its extension reaction. The blocking region may also be a polymerase enzyme blocking group, which is a group capable of blocking further elongation of a polymer. The blocking group may be any chemical group that can connect to a nucleotide, which allows 5' end of the labeled nucleotide to connect to 3' end of another nucleotide in a DNA strand, but does not allow a nucleotide to connect to a 3'-hydroxyl group of the labeled nucleotide. Properly, the absence of 3'OH group will prevent further extension by means of polymerase activity. In some embodiments, the blocking group is selected from acetyl, CH₃, glycyl, leucyl, and alanyl groups. In some other embodiments, the blocking group may be in a form of a dipeptide or tripeptide.

In some embodiments, the probe anchoring region (A) has a Tm value greater than that of the primer signal detection region (h), such that when the single-stranded amplification product (S1) binds to the probe, the probe anchoring region (A) of the single-stranded amplification product (S1) first specifically binds to the primer anchoring region (A') on the probe. Based on this, the complementary sequence of the primer signal detection region (h) of the single-stranded amplification product (S1) specifically binds to the probe signal detection region (H) on the probe, and then the 3' end of the single-stranded amplification product (S1) is extended (that is, first binding and then extending), such that the probe anchoring region (A) can play its auxiliary role in binding between the single-stranded amplification product (S1) obtained by the first amplification with the probe. In addition, the production of primer dimers during the amplification process can be effectively avoided. It should be noted that, even if the dimers are generated during the amplification process, the signal of the dimer may be distinguished via a melting curve; and if signal collection is performed at a higher melting temperature, the signal of the dimers will not be collected. A specific approach is as follows: the complementary sequence of the primer signal detection region (h) in the single-stranded amplification product (S1) specifically binds to the probe to form a double-stranded product, thereby resulting in a signal change; or, the complementary sequence of the primer signal detection region (h) in the single-stranded amplification product (S1) specifically binds to the probe and extends to form a secondary amplification double-stranded product (a double-stranded amplification product formed with the probe), thereby resulting in a different signal change. The difference in signal changes refers to different signal strengths and/or different melting temperatures.

In some embodiments, when the primer-probe composition comprises at least two second primers, the sequences of the primer signal detection regions (h) on different second primers are different, the complementary sequences of the primer signal detection regions (h) on different second primers are complementary to the sequences at different positions of the probe signal binding region (H); or, the positions at which the complementary sequences of the primer signal detection regions (h) on different second primers specifically bind to the probe signal binding region (H) are different. In some embodiments, the primer-probe composition comprises one first primer, two or more second primers, and one probe. In a specific example, the primer-probe composition comprises F1, R1, R2, R3, and P1. In this solution, a multiplex detection may be achieved with only one first primer and one probe. In some embodiments, the primer-probe composition comprises two or more first primers, two or more second primers, and two or more probes. In some embodiments, the primer-probe composition comprises at least one, at least two, at least three, at least four, or more primer pairs. In a specific example, the primer-probe composition comprises one primer pair, two primer pairs, three primer pairs, four primer pairs, five primer pairs, six primer pairs, seven primer pairs, eight primer pairs, or more primer pairs permitted by an amplification system. Those skilled in the art may understand that, different primer pairs are used to amplify different target sequences. In some embodiments, the primer-probe composition comprises at least one, at least two, at least three, at least four, or more probes. In a specific example, the primer-probe composition comprises one, two, three, four, five, six, seven, eight or more probes permitted by the amplification system. One or more probes are used to detect different target sequences.

As used herein, descriptions such as "one", "two", or "three" refer to that the type number of the probe or primer is "one", "two", or "three". The sequences and detection labels of different types of probes are different; and the sequences of different types of primers are different.

The expression 'the sequences of the primer signal detection regions (h) on different second primers are different' means that the sequences of the primer signal detection regions (h) of different second primers have different bases and/or lengths, such that different single-stranded pre-amplification products (S1) containing the complementary sequences of different primer signal detection regions (h) bind with the probe and extend to form different double-stranded amplification products formed with the probe, and the melting temperature curves of different double-stranded amplification products formed with the probe may be distinguished from each other. Since the melting temperature of the double-stranded amplification product formed with the probe is detected during melting curve analysis, the melting temperature of the double-stranded amplification product formed with the probe may be adjusted by varying the base and/or length of the sequence of the primer signal detection region (h).

In some embodiments, when the primer-probe composition comprises at least two probes, the sequences of the probe signal detection regions (H) of different probes are different, the sequences of the primer signal detection regions (h) of different second primers are different, and the positions at which the complementary sequences of the primer signal detection regions (h) specifically bind to the probe signal binding region (H) are different.

In the following, a nucleic acid detection process by using the primer-probe composition of the present application is exemplarily illustrated. By way of example, the target is a double-stranded target, specifically comprising a single-stranded target chain A and a single-stranded target chain B. wherein, the single-stranded target chain A comprises the first target sequence (X1), the second target sequence (X2), and a complementary sequence of the third target sequence (X3) thereon; the single-stranded target chain B comprises a complementary sequence of the first target sequence (X1), a complementary sequence of the second target sequence (X2), and the third target sequence (X3) thereon. The first primer comprises, from 5' end to 3' end, the probe anchoring region (A) and the first target sequence binding region (X1'); the second primer comprises, from 5' end to 3' end, the primer signal detection region (h), the second target sequence binding region (X2'), the complementary sequence (h') of the primer signal detection region, and the third target sequence binding region (X3'); and the probe comprises the primer anchoring region (A') and the probe signal detection region (H). When the primer-probe composition of the present application is used for nucleic acid detection, in the presence of a target, the second primer binds to the single-stranded target chain B, mainly by specifically binding the third target sequence binding region (X3') at 3' end of the second primer to the third target sequence (X3) on the single-stranded target chain B; subsequently, by taking the single-stranded target chain B as a template, the second primer extends, from its 3' end, to generate a complementary chain of single-stranded target chain B (also referred to as an amplification product of the second primer and target sequence); and the complementary chain (i.e. the amplification product of the second primer and the target sequence) is obtained by complementary base pairing with the single-stranded target chain B, and thus comprises the first target sequence (X1) and the second target sequence (X2). When the first primer is present, the first target sequence (X1) on the complementary chain specifically binds to the first target sequence binding region (X1') on the first primer, and the first primer is subjected to amplification to obtain the single-stranded amplification product (S1) by taking the amplification product of the second primer and the target sequence as a template. The single-stranded amplification product (S1) comprises, from 5' end to 3' end, the probe anchoring region (A), the first target sequence binding region (X1'), the complementary sequence of the second target sequence (X2), a complementary sequence (X3") of the third target sequence binding region (X3'), a complementary sequence of the complementary sequence (h') of the primer signal detection region, the complementary sequence (X2") of the second target sequence binding region (X2'), and the complementary sequence of the primer signal detection region (h).

In the single-stranded amplification product (S1), the Gibbs free energy of a conformation formed by the specific binding between the complementary sequence of the second target sequence (X2) and the complementary sequence(X2") of the second target sequence binding region (X2') is lower than the Gibbs free energy of a conformation formed by the specific binding between the complementary sequence of the primer signal detection region (h) and the complementary sequence of the complementary sequence (h') of the primer signal detection region; or, the Gibbs free energy of a conformation formed by the specific binding between the complementary sequence of the second target sequence (X2) and the complementary sequence (X2") of the second target sequence binding region (X2') is lower than the Gibbs free energy of a conformation formed by the specific binding between the complementary sequence of the primer signal detection region (h) and the complementary sequence (X2") of the second target sequence binding region (X2'). This solution ensures that when the single-stranded amplification product (S1) forms the stem-loop structure, the "stem" is formed by specific binding between the complementary sequence (X2") of the second target sequence (X2) and the complementary sequence (X2") of the second target sequence binding region (X2').

Further, in the above amplification process, if a specific amplification is performed, the single-stranded amplification product (S1) obtained will further form a stem-loop structure. Specifically, there are two complementary sequences in the single-stranded amplification product (S1), namely: 1) the complementary sequence of the primer signal detection region (h) specifically binds to the complementary sequence of the complementary sequence (h') of the primer signal detection region to form a stem structure; 2) the complementary sequence of the second target sequence (X2) specifically binds to the complementary sequence (X2") of the second target sequence binding region (X2') to form a stem structure. Therefore, the formed stem-loop secondary structure may have two conformations. In the present application, "tools capable of obtaining the secondary conformation of the single-stranded amplification product (S1), such as conformation prediction software or website" include, for example, ΔG online calculation website: https://sg.idtdna.com/calc/analyzer. When a conformation prediction is performed, the website may be used to predict the conformation of gene fragments and to calculate a Gibbs free energy change of a certain conformation. The "Gibbs free energy change", also referred to as Gibbs free energy or Gibbs function, is an important parameter in thermodynamics proposed by the renowned American physicist and chemist Gibbs in 1876; and is abbreviated as ΔG, and its unit can be kcal/mole. In the present application, the stability of the secondary structure is determined by the magnitude of ΔG. When the conformation of the single-stranded amplification product (S1) is predicted, an optional method is, for example, as follows: when the base composition of the obtained single-stranded amplification product (S1) is inputted into the tool capable of obtaining the secondary conformation of the single-stranded amplification product (S1), such as the conformation prediction software or the website, and the results show that the single-stranded amplification product (S1) can form a stem-loop secondary structure, and has a conformation of the stem-loop structure formed by specific binding between the complementary sequence of the second target sequence (X2) and the complementary sequence (X2") of the second target sequence binding region (X2'), it is the primer-probe composition required for nucleic acid detection.

In the present application, it is preferred that the stem-loop structure formed by specific binding between the complementary sequence of the second target sequence (X2) and the complementary sequence (X2") of the second target sequence binding region (X2') is the dominant conformation in the single-stranded amplification product (S1); that is, it is a preferred result of the present application that, during conformation prediction, there is almost no or no conformation in which "a stem structure is formed by specific binding between the complementary sequence of the primer signal detection region (h) and the complementary sequence (h') of the primer signal detection region". Therefore, when designing primers and probes, the design is selected wherein the formed single-stranded amplification product (S1) adopts, as its dominant conformation, a stem-loop structure formed by the binding of the second target sequence binding region (X2') and the second target sequence (X2), and such a design is used for nucleic acid detection. However, in the present application, if conformation prediction of the single-stranded amplification product (S1) simultaneously reveals the following two structures: "1) a stem structure formed by specific binding between the complementary sequence of the primer signal detection region(h) and the complementary sequence of the complementary sequence (h') of the primer signal detection region; 2) a stem-loop structure formed by specific binding between the complementary sequence of the second target sequence (X2) and the complementary sequence (X2") of the second target sequence binding region (X2')", it is also applicable to the nucleic acid detection of the present application as long as amplification enables the single-stranded amplification product (S1) adopting the conformation formed by the complementary sequence of the second target sequence (X2) and the complementary sequence (X2") of the second target sequence binding region (X2') to generate a sufficient detection signal.

After the single-stranded amplification product (S1) is formed, the 3' end of the single-stranded amplification product (S1) is in a released state. During the secondary amplification, if non-specific amplification is performed, the single-stranded amplification product (S1) obtained will not contain both the complementary sequence (X2") of the second target sequence binding region (X2') and the complementary sequence of the second target sequence (X2), and thus, a stem-loop structure will not be formed by the complementary sequence of the second target sequence (X2) and the complementary sequence (X2") of the second target sequence binding region (X2'); but in the product, there still exist the complementary sequence of the primer signal detection region (h) and the complementary sequence of the complementary sequence (h') of the primer signal detection region, and these two sequences bind to form a stem-loop structure, such that the 3' end of the single-stranded amplification product (S1) is in a closed state, making it difficult to be subjected to the next amplification that produces a detectable signal change. Therefore, the design of this structure achieves a stronger specific amplification. When the secondary amplification is performed, if the probe comprises, from 3' end to 5' end, the primer anchoring region (A') and the probe signal detection region (H), and a specific amplification is performed, the complementary sequence of the primer signal detection region (h) at 3' end of the single-stranded amplification product (S1) specifically binds to a partial sequence of the probe signal detection region (H) on the probe. At this point, amplification begins by taking the probe as a template and the single-stranded amplification product (S1) as a primer, and the 3' end of the single-stranded amplification product (S1) is extended by ≥ 0 bases to the 5' end of the probe, so as to obtain a double-stranded amplification product formed with the probe. If the probe comprises, from 5' end to 3' end, the primer anchoring region (A') and the probe signal detection region (H), the complementary sequence of the primer signal detection region (h) at 3' end of the single-stranded amplification product (S1) specifically binds to a partial sequence on the probe signal detection region (H), and at the same time, the probe anchoring region (A) at 5' end of the single-stranded amplification product (S1) specifically binds to the primer anchoring region (A') at 5' end of the probe; likewise, amplification begins by taking the probe as a template and the single-stranded amplification product (S1) as a primer, the 3' end of the single-stranded amplification product (S1) is extended, and after displacing the double-stranded binding of the probe anchoring region (A) and the primer anchoring region (A'), it continues to extend to the 5' end of the probe to obtain a double-stranded amplification product formed with the probe. Subsequently, the Tm value of the double-stranded amplification product formed with the probe is detected, so as to achieve detection.

When multiple target sequences are detected simultaneously, the complementary sequences of the primer signal detection regions (h) in different single-stranded amplification products (S1) obtained are different, such as a complementary sequence of the primer signal detection region (h1), a complementary sequence of the primer signal detection region (h2), etc.; and since the complementary sequences of different primer signal detection regions (h) bind to different positions of the probe respectively, the length and base composition of the finally obtained double-stranded amplification products formed with the probe are different, and the Tm values thereof are also different, thereby achieving multiplex detection. It should be understood that, in the present application, when entire sequences of the primer signal detection region (h) and the probe signal detection region (H) are identical, one probe can only bind to one target sequence; and when partial sequences of the primer signal detection region (h) and the probe signal detection region (H) are identical, multiple primer signal detection regions (h) may be designed. In this case, the complementary sequences of the primer signal detection regions (h) on different amplification products (S1) can specifically bind to the sequences at different positions of the probe signal detection region (H) respectively, thereby achieving simultaneous detection of multiple targets by using one probe. By designing the primer signal detection regions (h) and the probe signal detection regions (H) with different lengths and/or base compositions, the lengths and/or base compositions of the finally obtained double-stranded amplification products formed with the probe are different, so that different target sequences may be distinguished by detecting the Tm values of different products. Therefore, according to the method of the present application, simultaneous detection of multiple target sequences may be achieved by using only one probe. In addition, increasing the number of probes can further increase the number of targets detected simultaneously.

In addition, when the primer-probe composition of the present application is used for detection, the fluorescence change thereof is as follows: when a target sequence is not present, the probe is single-stranded, and the distance between the first detection group and the second detection group on the probe is relatively close, such that a detectable signal change will not be generated; and when a target sequence is present, the probe binds to the first primer, such that the distance between the first detection group and the second detection group on the probe is increased, thereby resulting in a detectable signal change.

Specifically, before the second specific amplification, when the probe does not bind to the single-stranded amplification product (S1), the probe is single-stranded, and since the probe has a curly shape due to molecular flexibility, the distance between the first detection group and the second detection group labelled on the probe is close, such that no detectable signal is generated; After the second specific amplification begins, the probe binds to the single-stranded amplification product (S1) to form a double-stranded structure, such that the distance between the first detection group and the second detection group labelled on the probe is increased, thereby resulting in a detectable signal change that can be detected by an instrument. After the specific amplification (i.e., the second specific amplification) is performed by taking the probe as a template and the single-stranded amplification product (S1) as a primer, the double-stranded amplification product formed with the probe is obtained. After the second specific amplification is completed, a melting curve analysis is performed, wherein during a process of temperature change, the amplification double-stranded product formed with the probe will melt into single-stranded state at a certain temperature, at this point, the distance between the first detection group and the second detection group changes, thereby generating a signal change (such as a change from the presence of fluorescent signal to absence of fluorescent signal, or weakening of a fluorescent signal) that can be detected by the instrument. The detection of the target sequence is finally achieved by measuring the Tm value of the double-stranded amplification product formed with the probe. By adjusting the sequence composition and length of the primer signal detection region (h) on the second primer, or a position at which the primer signal detection region (h) on the second primer is complementary to the probe signal detection region (H) of the probe, the Tm value of the obtained double-stranded amplification product formed with the probe can be adjusted, thereby achieving multiplex detection.

In some embodiments, the first linker sequence, the second linker sequence, the third linker sequence, the fourth linker sequence, and the fifth linker sequence each independently have a length of 0-20, 1-20, 5-20, 5-15, 10-15 bases, or any length range in a range of 0-20 bases. In a specific example, the first linker sequence, the second linker sequence, the third linker sequence, the fourth linker sequence, and the fifth linker sequence each independently have a length of 1, 2, 3, 4, 5, 6, 7, 8, 9, 10, 11, 12, 13, 14, 15, 16, 17, 18, 19, 20 bases, or any length in a range of 0-20 bases.

In some embodiments, the first primer and the second primer each independently have a length of 15-80, 20-50, 20-35, 35-70, 45-70, 55-65, or any length range in a range of 15-80 bases. In a specific example, the first primer and the second primer each are independently have a length of 15, 18, 20, 23, 25, 27, 30, 33, 35, 38, 40, 42, 45, 48, 50, 53, 55, 58, 60, 62, 65, 68, 70, 72, 75, 78, 80 bases, or any length in a range of 15-80 bases.

In some embodiments, the first target sequence binding region (X1') and the third target sequence binding region (X3') each independently have a Tm value of of 40-80 °C, 50-70 °C, 60-80 °C, 70-80 °C, or any temperature range in a range 40-80 °C. In a specific example, the first target sequence binding region (X1') and the third target sequence binding region (X3') each independently have a Tm value of 40, 45, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 °C, or any temperature in a range of 40-80 °C. In some embodiments, the third target sequence binding region (X3') has a GC content of 20-80%, 30-70%, 50-80%, 60-80%, 70-80%, 60-70%, 50-70%, 40-70%, 40-60%, 50-60%, 40-50%, or any content range in a range of 20-80%. In some embodiments, the first target sequence binding region (X1') has a length of 15-45, 15-25, 15-35, 25-45, 25-35, 35-45, or any length range in a range of 15-45 bases. In a specific example, the first target sequence binding region (X1') has a length of 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 bases, or any length in a range of 15-45 bases. In some embodiments, the third target sequence binding region (X3') has a length of 5-65, 5-15, 5-25, 5-35, 5-45, 5-55, 15-25, 15-35, 15-45, 5-55, 15-65, 25-35, 25-45, 25-55, 25-65, 35-45, 35-55, 35-65, 45-55, 45-65, 55-65 bases, or any length range in a range of 5-65 bases. In a specific example, the third target sequence binding region (X3') has a length of 5, 7, 10, 13, 15, 18, 20, 22, 25, 27, 30, 33, 35, 37, 40, 43, 45, 48, 50, 52, 55, 57, 60, 62, 64, 65 bases, or any length in a range of 5-65 bases.

In some embodiments, the primer signal detection region (h) and the complementary sequence (h') of the primer signal detection region each independently have a length of 5-65, 15-25, 15-35, 15-45, 5-55, 25-45, 25-55, 25-65, 35-45, 35-65, 45-65, 55-65 bases, or any length range in a range of 5-65 bases. In a specific example, the primer signal detection region (h) and the complementary sequence (h') of the primer signal detection region each independently have a length of 5, 7, 10, 13, 15, 18, 20, 22, 25, 27, 30, 33, 35, 37, 40, 43, 45, 48, 50, 52, 55, 57, 60, 62, 64, 65 bases, or any length in a range of 5-65 bases. In some embodiments, the primer signal detection region (h) and the complementary sequence (h') of the primer signal detection region each independently have a Tm value of 30-80 °C, 40-70 °C, 50-70 °C, 40-60 °C, or any temperature range in a range of 30-80 °C. In a specific example, the primer signal detection region (h) and the complementary sequence (h') of the primer signal detection region each independently have a Tm value of 30, 35, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 °C or any temperature in a range of 30-80 °C. In some embodiments, the primer signal detection region (h) and the complementary sequence (h') of the primer signal detection region each independently have a GC content of 20-80%, 50-80%, 50-70%, 40-60%, 50-60%, 40-50%, or any content range in a range of 20-80%.

In some embodiments, the second target sequence binding region (X2') has a length of 5-65, 15-45, 15-65, 25-35, 25-45, 35-45, 45-55, 55-65 bases, or any length range in a range of 5-65 bases. In a specific example, the second target sequence binding region (X2') has a length of 5, 7, 10, 13, 15, 18, 20, 22, 25, 27, 30, 33, 35, 37, 40, 43, 45, 48, 50, 52, 55, 57, 60, 62, 64, 65 bases, or any length in a range of 5-65 bases. In some embodiments, the second target sequence binding region (X2') has a Tm value of 40-80 °C, 40-70 °C, 60-70 °C, 50-60 °C, 40-50 °C, or any temperature range in a range of 40-80 °C. In a specific example, the second target sequence binding region (X2') has Tm value of 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 °C, or any temperature in a range of 40-80 °C. In some embodiments, the second target sequence binding region (X2') has a GC content of 40-80%, 60-70%, 50-70%, 50-60%, 40-50%, or any content range in a range of 40-80%.

In some embodiments, the probe has a length of 20-100 bases. In a specific example, the probe has a length of 20, 25, 30, 35, 40, 45, 50, 55, 60, 65, 70, 75, 80, 85, 90, 95, 100 bases, or any length in a range of 20-100 bases.

In some embodiments, the probe anchoring region (A) and primer anchoring region (A') each independently have a length of 5-35, 5-15, 5-25, 10-20, 10-30, 15-25, 15-35, 25-35 bases, or any length range in a range of 5-35 bases. In a specific example, the probe anchoring region (A) and primer anchoring region (A') each independently have a length of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 bases, or any length in a range of 5-35 bases. In some embodiments, the primer anchoring region (A') has a Tm value of 40-85 °C, 60-70 °C, 50-70 °C, 40-80 °C, 40-50 °C, or any temperature range in a range of 40-85 °C; for example, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 82, 84, 85 °C, or any temperature in a range of 40-85 °C. In some embodiments, the probe anchoring region (A) has a Tm value of 40-80 °C, 60-70 °C, 50-70 °C, 40-80 °C, 40-50 °C, or any temperature range in a range of 40-80 °C; for example, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 °C, or any temperature in a range of 40-80 °C. In some embodiments, the primer anchoring region (A') has a GC content of 20-80%, 50-80%, 60-80%, 40-70%, 40-60%, 50-60%, 40-50%, or any content range in a range of 20-80%. In some embodiments, the probe anchoring region (A) has a GC content of 30-80%, 50-80%, 60-70%, 50-70%, 40-70%, 40-60%, 50-60%, 40-50%, or any content range in a range of 30-80%.

In some embodiments, the probe signal binding region (H) has a length of 5-65, 15-45, 25-35, 25-45, 25-55, 35-55, 45-55 bases, or any length range in a range of 5-65 bases. In a specific example, the probe signal binding region (H) has a length of 5, 7, 10, 13, 15, 18, 20, 22, 25, 27, 30, 33, 35, 37, 40, 43, 45, 48, 50, 52, 55, 57, 60, 62, 64, 65 bases, or any length in a range of 5-65 bases. In some embodiments, the probe signal binding region (H) has a Tm value of 30-85 °C, 60-70 °C, 50-60 °C, 40-50 °C, or any temperature range in a range of 30-85 °C. In a specific example, the probe signal binding region (H) has a Tm value of 30, 35, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 85 °C, or any temperature in a range of 30-85 °C. In some embodiments, the probe signal binding region (H) has a GC content of 20-80%, 50-80%, 60-80%, 70-80%, 60-70%, 50-70%, 40-70%, 40-60%, 50-60%, 40-50%, or any content range in a range of 20-80%.

In some embodiments, the first detection group and the second detection group labelled on the probe generate a detectable signal change due to distance change. The generation of the signal change includes, but is not limited to: relying on fluorescence quenching (static or dynamic mechanism), fluorescence resonance energy transfer (FRET), and/or monomer-excimer emission switching with pyrene fluorophores. The processes of energy transfer, quenching, and excimer formation are dependent on the distance, such that when the probe binds to the target, the distance between the attached fluorescent groups changes due to structural rearrangement, which should lead to a change in the system signal.

In some embodiments, the first detection group is a fluorescence reporter group, and the second detection group is a fluorescence quencher group or a group that produces a fluorescence signal with the fluorescence reporter group via fluorescence resonance energy transfer. By way of example, the fluorescence reporter group optionally includes but is not limited to any one or more of ALEX-350, FAM, VIC, TET, CAL FluorGold540, JOE, HEX, CAL Fluor Orange 560, TAMRA, CAL Fluor Red 590, ROX, CAL Fluor Red 610, TEXAS RED, CAL Fluor Red 635, Quasar 670, Cy3, Cy5, Cy5.5 and Quasar 705; and the fluorescence quencher group includes but is not limited to any one or more of DABCYL, BHQ class (such as BHQ-1 or BHQ-2), ECLIPSE, and TAMRA. In some embodiments, the distance between the fluorescence reporter group and the fluorescence reporter group is 3-250 angstroms, 3-201 angstroms, 3-140 angstroms, or any distance range in a range of 3-250 angstroms. It should be noted that the positions of the first detection group and the second detection group on the probe in the present application are not particularly limited and may be labelled at any position on the probe. In addition, the labelling order of the first detection group and the second detection group on the probe is not limited, that is, the first detection group may be firstly labelled, followed by the second detection group, along the 5' to 3' end of the probe; or, the second detection group may be firstly labelled, followed by the first detection group, along the 5' end to 3' end of the probe. In some embodiments, when the probe comprises, from 5' end to 3' end, the primer anchoring region (A') and the probe signal detection region (H), the first detection group and the second detection group are labelled between the primer anchoring region (A') and the probe signal detection region (H). In some embodiments, when the probe comprises, from 3' end to 5' end, the primer anchoring region (A') and a probe signal detection region (H), the first detection group and the second detection group are labelled at 5' end of the probe signal detection region (H). In some embodiments, at least one detection group is labelled at 5' end of the probe signal detection region (H) of the probe. In some embodiments, at least one detection group is labelled within the probe signal detection region (H) of the probe.

The present application provides a primer-probe system for detection, comprising the primer-probe composition described above.

The present application further provides a nucleic acid detection method, comprising contacting a target sample with the primer-probe composition described above, performing at least two amplifications, and analyzing whether a target is present; wherein the target sample comprises a target sequence.

After the amplification is completed, a melting curve analysis is performed, wherein during a process of temperature change, the amplification double-stranded product (i.e. the double-stranded amplification product obtained by the second amplification) formed with the probe will be molten at a certain temperature, at this time, the probe with the first detection group and the second detection group will be changed from the double-stranded state to the single-stranded state, so as to cause a change in the distance between the detection groups, thereby generating a signal that can be detected by the instrument. The signals detected by the instrument are shown as a melting curve as a function of the change of the temperature, and when a Tm value at the peak position of the melting curve is within a characteristic Tm value range of the target in a specific detection channel, it indicates that the target is contained in the sample to be detected. The specific detection channel is a fluorescent channel corresponding to the first detection group on the probe, and the characteristic Tm value is a melting temperature of the double-stranded amplification product formed with the probe.

In the present application, "two amplifications" comprises: during a PCR reaction process, a first amplification in which "a primer specifically binds to a target to form a single-stranded pre-amplification product", and a second amplification in which "the single-stranded pre-amplification product specifically binds to the probe and undergoes amplification". The two amplifications may be performed in the same PCR reaction procedure, i.e., the denaturation temperature, annealing temperature and extension temperature used in the two amplifications are identical.

In the present application, there are multiple sets of first primer and second primer targeting different targets in the primer-probe composition. When the above primer probe composition is used for multiplex PCR detection, different target sequences can be identified by means of fluorescence channels or melting temperatures. For example, when different target sequences are identified by means of fluorescence channels, different probes may be used for different target sequences, and when different target sequences are identified by means of melting temperatures, a same probe may be shared by different target sequences.

In the present application, the target sample is not particularly limited, as long as it contains the target sequence. In some embodiments, the target sample comprises cells, tissues, organs, organisms obtained from living organisms or non-living sources, as long as the target sample contains the target sequence. In some embodiments, the target sample is a nucleic acid sample obtained from sources including but not limited to whole blood, plasma, serum, cerebrospinal fluid, urine, feces, cells, or tissues.

The method described herein is applicable to any suitable cell type. In some embodiments, the cells include but are not limited to bacteria, fungal cells, prokaryotes, protists, plant cells, animal cells, etc. In some embodiments, the fungal cells are, for example, yeast cells; and the animal cells, are, for example, mammalian cells, such as human cells. In some embodiments, the cells are of natural origin, such as cells isolated from tissue biopsy. In some embodiments, the cells are isolated from cell lines cultured in vitro. In some embodiments, the cells are derived from primary cell lines. In some embodiments, the cells are derived from immortalized cell lines. In some embodiments, the cells are genetically engineered cells.

In some embodiments, the source of the target sample includes any one or more of bacteria, viruses, and pathogens. The method of the present application is applicable to target samples derived from various types of bacteria, viruses, and pathogens. In some embodiments, the virus is a genetically engineered virus, a wild-type virus, and/or a modified virus. The method of the present application is applicable to various types of target samples, such as serum samples, plasma samples, whole blood samples, sputum samples, swab samples, lavage fluid samples, fresh tissue samples, and formalin-fixed paraffin-embedded tissues (FFPE).

In some embodiments, the amplification comprises the following steps:
randomly allocating a reaction system into 500 or more reaction units, wherein each reaction unit comprises a target of the sample to be detected or comprises no target of the sample to be detected;
and/or, performing PCR amplification on all the reaction units.

In some embodiments, the detection method is a digital PCR detection method.

In some embodiments, the reaction system is randomly allocated into 500-20000 reaction units, each containing a target of a sample to be detected or comprises no target of the sample to be detected.

In some embodiments, analyzing whether a target is present comprises the following steps:
obtaining a signal change generated by the amplification, and judging whether there is a target in the sample to be detected based on the signal change; and/or
heating a product obtained by the amplification, and judging whether there is a target contained in the sample to be detected based on a signal change generated before and after the heating.

In some embodiments, analyzing whether a target is present comprises the following steps:
obtaining a signal change generated by the amplification, and judging whether there is a target in the sample to be detected based on the signal change; and/or
performing melting curve analysis to a product obtained by the amplification, and judging whether there is a target contained in the sample to be detected.

In the present application, PCR may be adopted for sequence amplification.

In some embodiments, the PCR comprises pre-denaturation followed by denaturation, annealing, and extension. In some embodiments, a temperature for the pre-denaturation is 90-96 °C, 90-92 °C, 90-94 °C, 92-94 °C, 92-96 °C, 94-96 °C, or any temperature range in a range of 90-96 °C. In an example, a temperature for the pre-denaturation is 90 °C, 91 °C, 92 °C, 93 °C, 94 °C, 95 °C, 96 °C, or any temperature in a range of 90-96 °C. In some embodiments, a time for the pre-denaturation is 2-15 minutes, 5-15 minutes, 8-15 minutes, 12-15 minutes, 2-10 minutes, 2-8 minutes, 2-5 minutes, 8-12 minutes, 5-12 minutes, 12-15 minutes, or any time range in a range of 2-15 minutes. In an example, a time for the pre-denaturation is 2, 3, 4, 5, 6, 7, 8, 9, 10, 11, 12, 13, 14, 15 minutes, or any time in a range of 2-15 minutes.

In some embodiments, a temperature for the denaturation is 90-95 °C, 90-92 °C, 92-95 °C, or any temperature range in a range of 90-95 °C. In an example, a temperature for the pre-denaturation is 90 °C, 91 °C, 92 °C, 93 °C, 94 °C, 95 °C, or any temperature in a range of 90-95 °C. In some embodiments, a time for the denaturation is 10-60 seconds, 10-50 seconds, 10-40 seconds, 10-30 seconds, 10-20 seconds, 20-30 seconds, 20-40 seconds, 20-50 seconds, 20-60 seconds, 30-40 seconds, 30-50 seconds, 30-60 seconds, 40-50 seconds, 40-60 seconds, 50-60 seconds, or any time range in a range of 10-60 seconds. In an example, a time for the denaturation is 10, 15, 20, 25, 30, 35, 40, 45, 50, 55, 60 seconds, or any time in a range of 10-60 seconds.

In some embodiments, a temperature for the annealing is 50-75 °C, 50-60 °C, 50-70 °C, 55-65 °C, 55-75 °C, 60-70 °C, 65-75 °C, or any temperature range in a range of 50-75 °C. In an example, a temperature for the annealing is 50 °C, 52 °C, 54 °C, 56 °C, 58 °C, 60 °C, 62 °C, 64 °C, 66 °C, 68 °C, 70 °C, 72 °C, 74 °C, or any temperature in a range of 50-75 °C. In some embodiments, a time for the annealing is 30-90 seconds, 30-50 seconds, 40-80 seconds, 50-80 seconds, 60-70 seconds, or any time range in a range of 30-90 seconds. In an example, a time for the annealing is 30, 35, 40, 45, 50, 55, 60, 65, 70, 75, 80, 85, 90 seconds, or any time in a range of 30-90 seconds.

In some embodiments, the number of PCR cycles is 35-50, 35-40, 35-45, 40-45, 40-50, 45-50, or any range in a range of 35-50. By way of example, the number of PCR cycles is 35, 36, 37, 38, 39, 40, 41, 42, 43, 44, 45, 46, 47, 48, 49, 50.

In some embodiments, the PCR comprises performing pre-denaturation at 90-96 °C for 5-15 minutes, performing denaturation at 90-95 °C for 10-60 seconds, and performing annealing and extension at 50-75 °C for 30-90 seconds, for 35-50 cycles; and performing melting curve analysis at 35-95°C, with a heating rate of 0.05°C/s, and collecting fluorescence signal. By way of example, the PCR comprises performing pre-denaturation at 95 °C for 2 minutes; performing denaturation at 94 °C for 10 seconds, and performing annealing and extension at 56 °C for 30 seconds, for a total of 45 cycles; and performing melting curve analysis at 40-95°C, with a heating rate of 0.05°C/s, and collecting fluorescence signals.

In some embodiments, the first primer in the reaction system has a concentration of o30-1000 nM, 30-100 nM, 100-300 nM, 100-500 nM, 300-500 nM, 300-600 nM, 300-800 nM, 300-1000 nM, 50-800 nM, or any concentration range in a range of 30-1000 nM. In an example, the first primer in the reaction system has a concentration of 30 nM, 100 nM, 300 nM, 350 nM, 400 nM, 450 nM, 500 nM, 550 nM, 600 nM, 650 nM, 700 nM, 750 nM, 800 nM, 900 nM, 1000 nM, or any concentration in a range of 30-1000 nM.

In some embodiments, the second primer in the reaction system has a concentration of 30-500 nM, 30-50 nM, 50-100 nM, 50-150 nM, 50-200 nM, 50-300 nM, 50-400 nM, 100-500 nM, 300-500 nM, or any concentration range in a range of 30-500 nM. In an example, the second primer in the reaction system has a concentration of 30 nM, 50 nM, 80 nM, 100 nM, 150 nM, 200 nM, 250 nM, 300 nM, 350 nM, 400 nM, 450 nM, 500nM, or any concentration in a range of 30-500 nM.

In some embodiments, the probe in the reaction system has a concentration of 100-1200 nM, 150-250 nM, 150-400 nM, 150-650 nM, 150-800 nM, 150-1000 nM, 150-1200 nM, 250-650 nM, 300-500 nM, or any concentration range in a range of 100-1200 nM. In an example, the probe in the reaction system has a concentration of 100 nM, 150 nM, 200 nM, 250 nM, 300 nM, 350 nM, 400 nM, 450 nM, 500 nM, 600 nM, 700 nM, 800 nM, 900 nM, 1000 nM, 1200 nM, or any concentration in a range of 150-1200 nM.

In some embodiments, the first primer has a concentration of 30-1000 nM, the second primer has a concentration of 30-500 nM, and the probe has a concentration of 150-1200 nM. In a specific example, the first primer has a concentration of 500 nM, the second primer has a concentration of 100 nM, and the probe has a concentration of 400 nM.

The present application provides use of the primer-probe composition and/or primer-probe system described above, wherein the use comprises the preparation of a product for nucleic acid detection sand/or nucleic acid detection;
preferably, nucleic acid detection product is associated with a disease; and
more preferably, the disease is papilloma.

As understood by those skilled in the art, the detection method of the present application is applicable to any type of disease detection. The target may be selected from at least one of papillomavirus, adenovirus (ADV), influenza B virus (IBV), mycoplasma pneumoniae (MP), SARS-CoV-2, respiratory syncytial virus (RSV), influenza A virus (IAV), and non-small cell lung cancer. Examples of human papillomavirus include human papillomavirus type HPV16, human papillomavirus type HPV18, human papillomavirus type HPV56, and human papillomavirus type HPV58.

The present application further provides a kit for nucleic acid detection, comprising the primer-probe composition described above and/or the primer-probe system described above. In some embodiments, the kit further comprises an amplification reagent. In some embodiments, the amplification reagent comprises DNA polymerase and dNTPs. In some embodiments, when the nucleic acid template is RNA, the amplification reagent further comprises reverse transcriptase. In some embodiments, the amplification reagent also includes reagents that can promote PCR reactions, such as KCl, MgCl₂, Tris HCl, dithiothreitol (DTT), (NH₄)₂SO₄.

### Example

Hereafter, the specific examples of the present application will be described in more detail with reference to the accompanying drawings. Although specific examples of the present application are shown in the accompanying drawings, it should be understood that, the present application may be implemented in various forms and should not be limited by the examples set forth herein. Instead, these examples are provided to enable a more thorough understanding of the present application and to fully convey the scope of the present application to those skilled in the art.

### Example 1: Primer pairs and primer-probe composition for singleplex detection of human papillomavirus type HPV16

The primer pairs involved in this embodiment are as follows:
Primer F1, in 5' to 3' direction:
   TGTGGTGGGAAAGAGAACCTGAAACATTGCAGTTCTCTTTTGGTGC, SEQ ID NO:2; wherein, the TGTGGTGGGAAAGAG starting from the 5' end is a probe anchoring region (A), and the remaining portion AACCTGAAACATTGCAGTTCTCTTTTGGTGC is a first target sequence binding region (X1').
Primer R1, in 5' to 3' direction:
   CGATCGCCAAAGGGCGTAACCGAAATCGGTTGAACC, SEQ ID NO:3; wherein, the CGATCGCC starting from the 5' end is a primer signal detection region (h), the AAA following the primer signal detection region (h) is the linker sequence, and the GGGCGTAACCGAAATCGGTTGAACC following the linker sequence is a third target sequence binding region (X3').
Primer R2, in 5' to 3' direction:
   CGATCGCCCTGCTTTTATACTAACCGGGGCGATCGGGGCGTAACCGAAATCGGTT GAACC, SEQ ID NO:4; wherein, the CGATCGCC starting from the 5' end is a primer signal detection region (h), the CTGCTTTTATACTAACCGG following the primer signal detection region (h) is a second target sequence binding region (X2'), the GGCGATCG following the second target sequence binding region (X2') is a complementary sequence (h') of the primer signal detection region, and the GGGCGTAACCGAAATCGGTTGAACC following the complementary sequence (h') of the primer signal detection region is a third target sequence binding region (X3'). The primer signal detection region (h) in the primer R2 will spontaneously binds to the complementary sequence (h') of the primer signal detection region to form a first stem-loop structure. When the first stem-loop structure exists stably (i.e., as the dominant conformation), the Gibbs free energy change of this secondary structure is calculated as ΔG1, and the ΔG1 value is -18.04 kcal/mole, representing the most dominant conformation. ΔG values of different secondary structures may be exemplarily obtained through a ΔG online-calculation website: https://sg.idtdna.com/calc/analyzer.
Primer R3, in 5' to 3' direction:
   CCGGTTAGCTGCTTTTATACTAACCGGAAAGGGCGTAACCGAAATCGGTTGAACC, SEQ ID NO:5; wherein, the CCGGTTAG starting from the 5' end is a primer signal detection region (h), the CTGCTTTTATACTAACCGG following the primer signal detection region (h) is a second target sequence binding region (X2'), the AAA following the second target sequence binding region (X2') is a linker sequence, and the GGGCGTAACCGAAATCGGTTGAACC following the linker sequence is the third target sequence binding region (X3').

The primer-probe composition involved in this example comprises the primer pairs described above, and a probe P, as shown below:
Probe P, in 5' to 3' direction:
CTCTTTCCCACCACATTTAGCCGACCGTAGTCCGGTTAGCCTAACCGGCGATCGC C, SEQ ID NO:1; wherein, the CTCTTTCCCACCACA starting from the 5' end is a primer anchoring region (A'), and the remaining part following the primer anchoring region (A') is a probe signal detection region (H). The sequence of CGATCGCC at the 3' end of the probe signal detection region (H) of the probe is the same as the sequence of the primer signal detection regions (h) of the primer R2 and primer R1; and the sequence of the CCGGTTAG adjacent to CGATCGCC at the 3' end of the probe signal detection region (H) of the probe is the same as the sequence of the primer signal detection region (h) of the primer R3. Probe labelling: T16-CY5, T28-BHQ2 (starting from 5' end, labelling the 16th base T with CY5; and labelling the 28th base T with BHQ2).

When a specific amplification is performed on the pathogen target, the forward primer F1 and the reverse primer (primer R1 or R2 or R3) amplify the target nucleic acid to obtain a double-stranded pre-amplification sub-product. A single-stranded amplification product (S1) obtained by amplifying with the primer R2 comprises, from 5' end to 3' end, the probe anchoring region (A), the first target sequence binding region (X1'), a complementary sequence of the second target sequence (X2), a complementary sequence of the third target sequence binding region (X3'), a complementary sequence of the complementary sequence (h') of the primer signal detection region, a complementary sequence of the second target sequence binding region (X2'), and the complementary sequence of the primer signal detection region (h) in sequence. The complementary sequence (X2") of the second target sequence binding region (X2') and the complementary sequence of the second target sequence (X2) in the single-stranded amplification product (S1) spontaneously binds to to form a second stem-loop structure. When the second stem-loop structure exists stably (i.e., as the dominant conformation), the Gibbs free energy change of this secondary structure is calculated as ΔG2, the ΔG2 value is -32.98 kcal/mole, representing the most dominant conformation. ΔG values of different secondary structures may be exemplarily obtained through a ΔG online-calculation website: https://sg.idtdna.com/calc/analyzer.

The probe signal detection region (H) of probe P specifically binds to the complementary sequence of the primer signal detection region (h) of one single-stranded amplification product (S1), and the primer anchoring region (A') of the probe P is complementarily paired to the probe anchoring region (A) at 5' end of the single-stranded amplification product (S1). After amplification, a melting curve analysis is performed, wherein, during a process of temperature change, the amplification double-stranded product formed by the probe P and the single-stranded amplification product (S1) will melt at a certain temperature, at this time, the probe P labeled with the first detection group and the second detection group will revert to the single-stranded state, thereby generating a change in fluorescence signal that can be detected by an instrument.

### Example 2: Singleplex detection system and method for human papillomavirus Type HPV16

The singleplex detection system for human papillomavirus type 16 (HPV16) comprises the primer-probe composition of Example 1, a template, a DNA polymerase, a UDG enzyme, and a reaction buffer (a specific PCR reaction system is shown in Table 1), and is used for target-specific amplification and fluorescence signal detection.

A detection method comprises the following steps:
(1) Firstly, during PCR amplification cycles, the forward primer F1 and the reverse primer (R1, R2, or R3) can specifically amplify a target nucleic acid sequence to obtain a single-stranded amplification product (S1); and the single-stranded amplification product (S1) specifically binds to probe P and extends by ≥ 0 bases, to form a double-stranded amplification product.
(2) After the PCR amplification is completed, melting curve analysis is performed, in which the amplification double-stranded product formed by the probe P and the single-stranded amplification product (S1) will melt at a certain temperature, at this time, the probe P labeled with the first detection group and the second detection group will revert to the single-stranded state, thereby generating a change in fluorescence signal that can be detected by an instrument.

The reaction system in this example is shown in Table 1:

**Table 1. PCR reaction system**

| Reagent components | | Concentration |
|---|---|---|
| Singleplex detection system for human papillomavi rus type HPV16 | 2×PCR Reaction Buffer | 1× |
| | DNA Polymerase | 3 U |
| | Probe (P1) | 400 nM |
| | Forward Primer (F1) | 500 nM |
| | Reverse primer (R1 or R2 or R3) | 100 nM |
| | Nucleic acid template | 50 copies |
| | Ultrapure water | Adding to 25 µ L |

The reaction conditions for PCR in this example were as follows: performing pre-denaturation at 95 °C for 2 minutes; performing denaturation at 94 °C for 10 seconds, and performing annealing and extension at 56 °C for 30 seconds, for a total of 45 cycles; and performing melting curve analysis at 40-95 °C, with a heating rate of 0.05 °C/s, and collecting fluorescence signals.

In this example, the reaction buffer and a primer-probe premixed solution were mixed according to the reaction system described in Table 1. The nucleic acid template to be detected was a human papillomavirus Type HPV16 plasmid synthesized by General Biosystems (Anhui) Co., Ltd.. The nucleic acid template was quantified and diluted, and mixed with the reaction system at a ratio of 50 copies/reaction; and at the same time, a no-template control (NTC) (obtained by replacing the nucleic acid template with ultrapure water or 1×TE buffer) was prepared, and then a PCR amplification and a melting curve analysis were performed. The presence or absence of Type HPV16 in the sample can be judged based on a characteristic Tm value of the target in a specific detection channel.

For detection, illustratively, this example uses the SLAN real-time fluorescence quantitative PCR instrument and reagent consumables from Shanghai Hongshi Medical Technology Co., Ltd. The 2x PCR reaction buffer comprises: 3 mM MgCl₂, 30 mM Tris-HCl with pH 8.3, 0.5 mM dNTP, 70 mM (NH₄)₂SO₄, etc. Compatible with the detection instrument, the detection results of the kit described in this example were analyzed by using an analysis software of the SLAN real-time fluorescence quantitative PCR instrument from Shanghai Hongshi Medical Technology Co., Ltd.

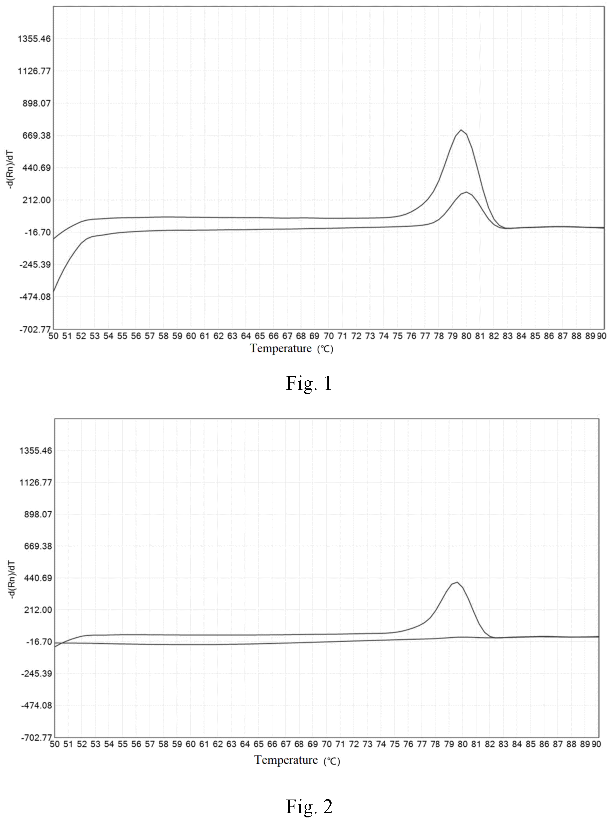

The results were interpreted based on the Tm value of the melting curve. In the PCR reaction system, except for the reverse primers, all other components used in Figures 1 to 3 (such as probes, forward primers, etc.) were the same.

Fig. 1 shows a melting curve of an amplification product obtained by using the reverse primer (R1), wherein the melting curve with a higher melting peak is the one with the HPV16 plasmid added as the nucleic acid template, and the melting curve with a lower melting peak is the one of the no-template control (NTC). It can be seen from Fig. 1 that a false positive melting peak also appears in the no-template control well, and it has the same position as the melting peak with a Tm value of around 80 °C formed in the CY5 channel by the positive reaction well with addition of the HPV16 plasmid, indicating that the use of a primer having a structure similar to that of the reverse primer R1 may easily lead to false positive problem.

Fig. 2 shows a melting curve of an amplification product obtained by using the reverse primer (R2), wherein the melting curve having a melting peak is the one with HPV16 plasmids added as the nucleic acid template, and the melting curve having no melting peak is the one of the no-template control (NTC). It can be seen from Fig. 2 that a melting peak with a Tm value of 79.5°C is formed in a CY5 channel by the positive reaction well with addition of the HPV16 plasmid, while no melting peak is formed by the no-template control well, indicating that the use of a primer having a structure similar to that of the reverse primer R2 may avoid the false positive problem.

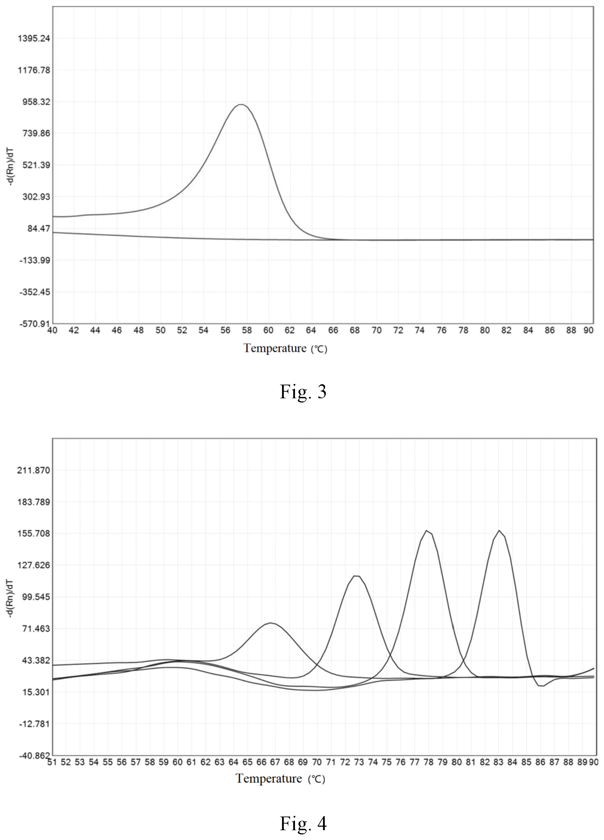

Fig. 3 shows a melting curve of an amplification product obtained by using the reverse primer (R3), wherein the melting curve having a melting peak is the one with HPV16 plasmids added as the nucleic acid template, and the melting curve having no melting peak is the one of the no-template control (NTC). It can be seen from Fig. 3 that a melting peak with a Tm value of 57°C is formed in a CY5 channel by the positive reaction well with addition of the HPV16 plasmid, while no melting peak is formed by the no-template control well, indicating that the use of a primer having a structure similar to that of the reverse primer R3 may avoid the false positive problem.

In addition, it can be seen from Fig. 2 and Fig. 3 that, the reverse primers R2 and R3 have the same target sequence binding region (GGGCGTAACCGAAATCGGTTGAACC), but their primer signal detection regions (CGATCGCC in R2; and CCGGTTAG in R3) are different, such that the positions at which the single-stranded amplification products formed by each of the two and the same probe P1 are different, thereby leading to a significant difference in the Tm values of the melting peak (79.5 °C in Fig. 2; and 57 °C in Fig. 3).

### Example 3: Multiplex detection system and method

This example involves multiplex detection for human papillomavirus types HPV16, HPV18, HPV56, and HPV58. The primers and probe involved are as follows (in 5' to 3' direction):

### Primers:

PF1/HPV16:GCGCCGCTCGCGGGCGTAACCGAAATCGGTTGAACCGAAACCGG, SEQ ID NO:6; wherein, the GCGCCGCTCGC starting from the 5' end is a probe anchoring region (A).

PR1/HPV16:ACGGCGATGCAGACATTTTATGCACCAAAAGAATCGCCGTCGCTCCT GTGGGTCCTGAAACATTGCA, SEQ ID NO:7; wherein, the ACGGCGA starting from the 5' end is a primer signal detection region (h), and the TCGCCGT starting from the 34^{th} base at 5' end is the complementary sequence (h') of the primer signal detection region.

PF2/HPV18:GCGCCGCTCGCCACAATACTATGGCGCGCTTTGAGGATCC, SEQ ID NO:8; wherein, the GCGCCGCTCGC starting from the 5' end is a probe anchoring region (A).

PR2/HPV18:TCGCTACTAACACGGCGACCCTACAAGCTACCTGTAGTAGCGAGCA GTGAAGTGTTCAGTTCCGTGCACA, SEQ ID NO:9; wherein, the TCGCTACT starting from the 5' end is a primer signal detection region (h), and the TCGCCGT starting from the 36^{th} base at the 5' end is a complementary sequence (h') of the primer signal detection region.

PF3/HPV56:GCGCCGCTCGCTGTGGACATATCCATGGAGCCACAATTCAA, SEQ ID NO:10; wherein, the GCGCCGCTCGC starting from the 5' end is a probe anchoring region (A).

PR3/HPV56:AGCGCGATTCCACAGGAACGTCCACGAAGCCTATCGCGCTCAATTA AAGGTATTTCTAATACCTCACTCAAGTGG, SEQ ID NO:11; wherein, the AGCGCGA starting from the 5' end is a primer signal detection region (h), and the TCGCGCT starting from the 34^{th} base at 5' the end is a complementary sequence (h') of the primer signal detection region.

PF4/HPV58:GCGCCGCTCGCTGGTAGGCTACTGCAGGACTATGTTCC, SEQ ID NO:12; wherein, the GCGCCGCTCGC starting from the 5' end is a probe anchoring region (A).

PR4/HPV58:TCGTACGGGGACGCAGAGGAGAAACCACGGACCCGTACGATCTCCA ACGCCTGACACAAATCATGCA, SEQ ID NO:13; wherein, the TCGTACGG starting from the 5' end is a primer signal detection region (h), and the CCGTACGA starting from the 33^{rd} base at the 5' end is a complementary sequence(h') of the primer signal detection region.

Probe PB: GCGAGCGGCGCTACGGCGATCGCTACTAGCGCGATCGTACGG (labelling: T12-FAM, T20-BHQ1), SEQ ID NO: 14. The GCGAGCGGCGC starting from the 5' end of the probe is a primer anchoring region (A'), and the remaining part following the primer anchoring region (A') is a probe signal detection region (H); wherein, the ACGGCGA following the primer anchoring region (A') is identical to the sequence of the primer signal detection region (h) in PR1/HPV16; the subsequent TCGCTACT is identical to the sequence of the primer signal detection region (h) in PR2/HPV18; the subsequent AGCGCGA is identical to the sequence of the primer signal detection region (h) in PR3/HPV56; and the final TCGTACGG is identical to the sequence of the primer signal detection region (h) in PR4/HPV58.

The multiple detection system for human papillomavirus in this example comprises the primer-probe composition described above, a template, a DNA polymerase, a UDG enzyme, and a reaction buffer (a specific PCR reaction system is shown in Table 2), and is used for target-specific amplification and fluorescence signal detection.

A detection method comprises the following steps:
(1) Firstly, during PCR amplification cycles, after amplification, a single-stranded amplification product (S1) is obtained; and the single-stranded amplification product (S1) specifically binds to probe P and extends by ≥ 0 bases, to form a double-stranded amplification product.
(2) After the PCR amplification is completed, melting curve analysis is performed, in which the amplification double-stranded product formed by the probe P and the single-stranded amplification product (S1) will melt at a certain temperature, at this time, the probe P1 with the first detection group and the second detection group will revert to the single-stranded state, thereby generating a signal change that can be detected by an instrument.

The reaction system in this example is shown in Table 2:

**Table 2. PCR reaction system**

| Reagent components | | Concentration |
|---|---|---|
| | 2×PCR Reaction Buffer | 1× |
| Singleplex detection system for human papillomavi rus type HPV16 | DNA Polymerase | 3 U |
| | Probe (PB) | 400 nM |
| | Forward primers (PF1/HPV16, PF2/HPV18, PF3/HPV56, PF4/HPV58) | 500 nM each |
| | Reverse primers (PR1/HPV16, PR2/HPV18, PR3/HPV56, PR4/HPV58) | 100 nM each |
| | Nucleic acid template (HPV16 or HPV18 or HPV56 or HPV 58 or 1X TE Buffer) | 50 copies |
| | Ultrapure water | Adding to 25 µ L |

The reaction conditions for PCR in this example were as follows: performing pre-denaturation at 95 °C for 2 minutes; performing denaturation at 94 °C for 10 seconds, and performing annealing and extension at 56 °C for 30 seconds, for a total of 45 cycles; and performing melting curve analysis at 40-95 °C, with a heating rate of 0.05 °C/s, and collecting fluorescence signals.

In this example, the reaction buffer and a primer-probe premixed solution were mixed according to the reaction system described in Table 2. The nucleic acid template to be detected was a human papillomavirus type HPV16 plasmid, a human papillomavirus type HPV18 plasmid, a human papillomavirus type HPV56 plasmid, or a human papillomavirus type HPV58 plasmid, all of which were synthesized by General Biosystems (Anhui) Co., Ltd.. The nucleic acid template was quantified and diluted, and mixed with the reaction system at a ratio of 50 copies/reaction; and at the same time, a no-template control (NTC) (obtained by replacing the nucleic acid template with ultrapure water or 1×TE buffer) was prepared, and then a PCR amplification and a melting curve analysis were performed. The presence or absence of the virus in the sample can be judged based on a characteristic Tm value of the target in a specific detection channel.

For detection, illustratively, this example uses the SLAN real-time fluorescence quantitative PCR instrument and reagent consumables from Shanghai Hongshi Medical Technology Co., Ltd. The 2× PCR reaction buffer comprises: 3 mM MgCl2, 30 mM Tris-HCl with pH 8.3, 0.5 mM dNTP, 70 mM (NH4)2SO4, etc. Compatible with the detection instrument, the detection results of the kit described in this example were analyzed by using the analysis software of the SLAN real-time fluorescence quantitative PCR instrument from Shanghai Hongshi Medical Technology Co., Ltd.

The results were interpreted based on the Tm value of the melting curve.

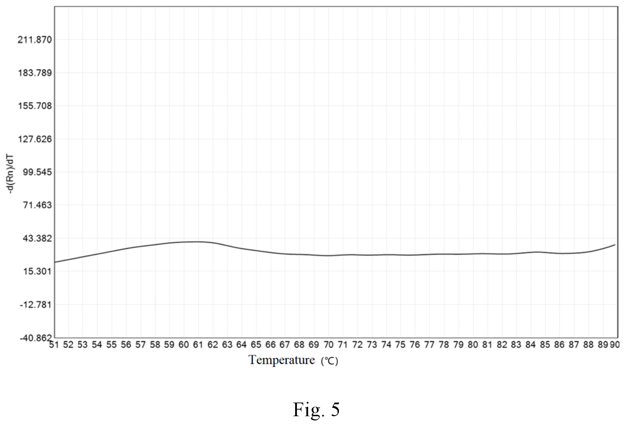

All the primers and probe of this example were added, with only the template of one virus added at a time; and after PCR amplification, the double-stranded amplification products formed with the probe were subjected to melting curve analysis, and the melting curves of the four targets were plotted on one graph, as specifically shown in Fig. 4. From left to right in Fig. 4 were the melting peaks of positive reaction wells with addition of the type HPV16 plasmid, HPV18 plasmid, HPV56 plasmid, and HPV58 plasmid respectively. All the primers and probe of this example were added, without the addition of positive plasmid template (i.e., no-template control); and then the double-stranded amplification products formed with the probe were subjected to melting curve analysis (i.e., the NTC group), and the results were specifically shown in Fig. 5. Fig. 5 indicates that none of the primers cause the primer dimers.

The description in the present disclosure is only provided for exemplary and descriptive purposes, but is not exhaustive or intended to limit the present disclosure to the form disclosed. Many modifications and changes are obvious to one of ordinary skill in the art. The examples are selected and described to better illustrate the principles and practical applications of the present disclosure, and to enable one of ordinary skill in the art to understand the present disclosure, so as to design various examples with various modifications suitable for specific uses.

## Claims

1. A primer-probe composition for nucleic acid detection, comprising at least one first primer, at least one second primer, and at least one probe;
the first primer comprises, from 5' end to 3' end, a first target sequence binding region (X1');
the second primer comprises, from 5' end to 3' end, a primer signal detection region (h), a second target sequence binding region (X2'), and a third target sequence binding region (X3'); when a target is not present, the primer signal detection region (h) specifically binds to a partial sequence of the second primer to form a first stem-loop structure;
wherein, the first target sequence binding region (X1') and the second target sequence binding region (X2') specifically bind to a first target sequence (X1) and a second target sequence (X2) of one target chain in a double-stranded target respectively, and the third target sequence binding region (X3') can specifically bind to a third target sequence (X3) of the other target chain in the double-stranded target;
the probe comprises a probe signal detection region (H) and a first detection group and a second detection group labeled at any position on the probe, wherein the first detection group and the second detection group generate a detectable signal change through a change in distance between them on the probe; and
a complementary sequence of the primer signal detection region (h) specifically binds to the probe signal detection region (H).

2. The primer-probe composition of claim 1, wherein, when a target is not present, the partial sequence of the second primer that specifically binds to the primer signal detection region (h) to form the first stem-loop structure is a complementary sequence (h') of the primer signal detection region, which is located between the second target sequence binding region (X2') and the third target sequence binding region (X3');
the primer signal detection region (h), the complementary sequence (h') of the primer signal detection region, and the probe are not complementary and/or identical to any target sequence; and
preferably, the second primer further comprises a third linker sequence located between the complementary sequence (h') of the primer signal detection region and the third target sequence binding region (X3'), wherein the third linker sequence is not complementary and/or identical to any target sequence.

3. The primer-probe composition of claim 1, wherein, when a target is not present, the partial sequence of the second primer that specifically binds to the primer signal detection region (h) to form the first stem-loop structure is the second target sequence binding region (X2'), and the second primer further comprises a third linker sequence located between the second target sequence binding region (X2') and the third target sequence binding region (X3'), wherein the third linker sequence is not complementary and/or identical to any target sequence.

4. The primer-probe composition of any one of claims 1-3, wherein any two of the first target sequence binding region (X1'), the third target sequence binding region (X3'), and the second target sequence binding region (X2') are not complementary or identical in sequence.

5. The primer-probe composition of any one of claims 1-4, wherein the first primer further comprises a probe anchoring region (A) located at 5' end of the first target sequence binding region (X1');
and/or, the probe further comprises a primer anchoring region (A'); and
the primer anchoring region (A') specifically binds to the probe anchoring region (A).

6. The primer-probe composition of any one of claims 1-5, wherein the probe comprises, from 5' end to 3' end, the probe signal detection region (H) and the primer anchoring region (A');
or, the probe comprises, from 3' end to 5' end, the probe signal detection region (H) and the primer anchoring region (A').

7. The primer-probe composition of any one of claims 1-6, wherein a Tm value of the probe anchoring region (A) is greater than a Tm value of the primer signal detection region (h).

8. The primer-probe composition of any one of claims 5-7, wherein the first primer further comprises a fourth linker sequence located between the probe anchoring region (A) and the first target sequence binding region (X1');
and/or, the second primer further comprises a first linker sequence located between the primer signal detection region (h) and the second target sequence binding region (X2'), and/or, a second linker sequence located between the second target sequence binding region (X2') and the complementary sequence (h') of the primer signal detection region;
and/or, the probe further comprises a fifth linker sequence located between the primer anchoring region (A') and the probe signal detection region (H); and
the first linker sequence, the second linker sequence, the fourth linker sequence, and the fifth linker sequence each are not complementary and/or identical to any target sequence.

9. The primer-probe composition of any one of claims 1-8, wherein a blocking region is located at 3' end of the probe.

10. The primer-probe composition of any one of claims 1-9, wherein, when the primer-probe composition comprises one probe and at least two said second primers, at least sequences of the primer signal detection regions (h) on different said second primers are different, and positions where the complementary sequences of the primer signal detection regions (h) on different said second primers specifically bind to the probe signal binding region (H) are different; and
preferably, one, two or more first primers are included.

11. A primer-probe system for nucleic acid detection, comprising the primer-probe composition of any one of claims 1-10.

12. A nucleic acid detection method, comprising contacting a target sample with the primer-probe composition of any one of claims 1-10 and/or the primer-probe system of claim 11, performing at least two amplifications, and analyzing whether a target is present;
wherein, the target sample includes a target;
preferably, a source of the target sample includes any one or more of bacteria, viruses, and pathogens;
preferably, a method for analyzing whether a target is present comprises: determining whether the target is present by a signal change before and after amplification; and/or, determining whether the target is present by a melting curve anylsis of an amplification product; and/or, determining whether the target is present by a signal change before and after denaturation of the amplification product; and
preferably, the amplification comprising: randomly allocating a reaction system to at least 500 reaction units, wherein each reaction unit comprises a target of a sample to be detected or comprises no target of the sample to be detected;
and/or, performing PCR amplification on all the reaction units.

13. The method of claim 12, wherein after the target sample is contacted with the primer-probe composition and/or the primer-probe system, when the target is present in the target sample, a first specific amplification is performed by taking the target as a template and the first primer and second primer as primers to obtain a single-stranded amplification product (S1); wherein, the single-stranded amplification product (S1) comprises, from 5' end to 3' end, at least the first target sequence binding region (X1'), a complementary sequence of the second target sequence (X2), a complementary sequence (X3") of the third target sequence binding region (X3'), optionally a complementary sequence of the third linker sequence and/or a complementary sequence of the complementary sequence (h') of the primer signal detection region, a complementary sequence (X2") of the second target sequence binding region (X2'), and a complementary sequence of the primer signal detection region (h);
in the single-stranded amplification product (S1), a Gibbs free energy of a conformation formed by specific binding of the complementary sequence of the second target sequence (X2) and the complementary sequence (X2") of the second target sequence binding region (X2') is lower than a Gibbs free energy of a conformation formed by specific binding of the complementary sequence of the primer signal detection region (h) and the complementary sequence of the complementary sequence (h') of the primer signal detection region; or, the Gibbs free energy of the conformation formed by specific binding of the complementary sequence of the second target sequence (X2) and the complementary sequence (X2") of the second target sequence binding region (X2') is lower than a Gibbs free energy of conformation formed by specific binding of the complementary sequence of the primer signal detection region (h) and the complementary sequence (X2") of the second target sequence binding region (X2');
the single-stranded amplification product (S1) forms a second stem-loop structure by binding the complementary sequence of the second target sequence to the complementary sequence (X2") of the second target sequence binding region (X2');
when the target is present, the second stem-loop structure of the single-stranded amplification product (S1) is melt, and a second specific amplification is performed by taking the probe as a template and the single-stranded amplification product (S1) as a primer to obtain a double-stranded amplification product formed with the probe; and
a melting temperature of the double-stranded amplification product formed with the probe is detected to achieve nucleic acid detection.

14. Use of the primer-probe composition of any one of claims 1-11 and/or the primer-probe system of claim 12, comprising the preparation of a product for nucleic acid detection and/or nucleic acid detection;
preferably, the product for nucleic acid detection is associated with a disease; and
more preferably, the disease is papilloma.

15. A kit for nucleic acid detection, comprising the primer-probe composition of any one of claims 1-10 and/or the primer-probe system of claim 11;
preferably, the kit further comprises an amplification reagent;
more preferably, the amplification reagent comprises DNA polymerase and dNTPs; and
more preferably, the amplification reagent further comprises a reverse transcriptase.
